(19) Europäisches Patentamt
European Patent Office
Office européen des brevets

(11) Publication number: **0 037 080**
**B1**

(12) **EUROPEAN PATENT SPECIFICATION**

(45) Date of publication of patent specification: **13.03.85**

(21) Application number: **81102268.0**

(22) Date of filing: **26.03.81**

(51) Int. Cl.⁴: **C 07 D 487/04,**
C 07 D 205/08, C 07 F 7/10 //
(C07D487/04, 209/00, 205/00)

(54) **4-(3-carboxy-2-oxopropyl)-azetidino-2-ones and process for their preparation.**

(30) Priority: **27.03.80 US 134408**

(43) Date of publication of application:
**07.10.81 Bulletin 81/40**

(45) Publication of the grant of the patent:
**13.03.85 Bulletin 85/11**

(84) Designated Contracting States:
**CH DE FR GB LI NL**

(56) References cited:
**EP-A-0 000 828**
**EP-A-0 007 973**
**DE-A-2 811 514**

(73) Proprietor: **MERCK & CO. INC.**
**126, East Lincoln Avenue P.O. Box 2000**
**Rahway New Jersey 07065 (US)**

(72) Inventor: **Christensen, Burton G.**
**195 Watchung Terrace**
**Scotch Plains New Jersey 07076 (US)**
Inventor: **Ratcliffe, Ronald W.**
**234 Matawan Avenue**
**Matawan New Jersey 07747 (US)**
Inventor: **Salzmann, Thomas N.**
**387 Brook Avenue**
**North Plainfield New Jersey 07062 (US)**

(74) Representative: **Abitz, Walter, Dr.-Ing. et al**
**Abitz, Morf, Gritschneder P.O. Box 86 01 09**
**D-8000 München 86 (DE)**

**EP 0 037 080 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to 4-(3-carboxy-2-oxopropyl)-azetidino-2-ones selected from

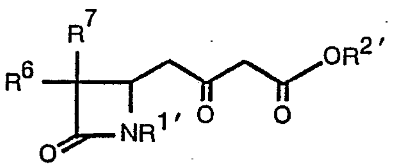

and

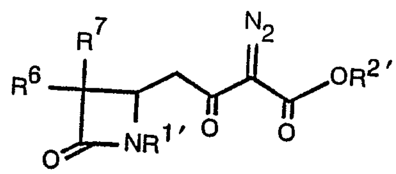

wherein $R^{1'}$ and $R^{2'}$ are hydrogen or a readily removable protecting group; and wherein $R^6$ and $R^7$ are independently selected from hydrogen; substituted and unsubstituted: alkyl, alkenyl, and alkynyl, having from 1—10 carbon atoms; cycloalkyl, cycloalkylalkyl, and alkylcycloalkyl, having 3—6 carbon atoms in the cycloalkyl ring and 1—6 carbon atoms in the alkyl moieties; phenyl; aralkyl, aralkenyl, and aralkynyl wherein the aryl moiety is phenyl and the aliphatic portion has 1—6 carbon atoms; heteroaryl, heteroaralkyl, heterocyclyl and heterocyclylalkyl; wherein the substituent or substituents relative to the above-named radicals are selected from:

chloro, bromo, fluoro,

$-OR^1$, $-NH_2$,

$-OH$, $-NR^1R^2$,

$-O\overset{O}{\overset{\|}{C}}NR^1R^2$, $-NHR^1$, $-\overset{O}{\overset{\|}{C}}NR^1R^2$, 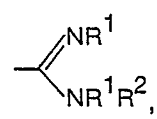

$-SO_2NR^1R^2$, $-NH\overset{O}{\overset{\|}{C}}NR^1R^2$, $-R^2N\overset{O}{\overset{\|}{C}}R^1$, $-CO_2H$,

$-CO_2R^1$, $-\overset{O}{\overset{\|}{C}}R^1$, $-O\overset{O}{\overset{\|}{C}}R^1$, $-SH$,

$-\overset{O}{\overset{\|}{S}}R^1$, $-\underset{O}{\underset{\|}{\overset{O}{\overset{\|}{S}}}}R^1$, $-CN$, and $-N_3$

wherein, relative to the above listed substituents on $R^6$, and $R^7$, the groups $R^1$ and $R^2$ are independently selected from: hydrogen, alkyl, alkenyl, and alkynyl, having from 1—10 carbon atoms; cycloalkyl, cycloalkylalkyl, and alkylcycloalkyl, having 3—6 carbon atoms in the cycloalkyl ring and 1—6 carbon atoms in the alkyl moieties; phenyl; aralkyl, aralkenyl, and aralkynyl wherein the aryl moiety is phenyl and the aliphatic portion has 1—6 carbon atoms; heteroaryl, heteroaralkyl, heterocyclyl and heterocyclylalkyl; and

wherein the above-named heterocyclic moieties defined for $R^6$, $R^7$, $R^1$ and $R^2$ are derived from the following alkylating or acylating agents.

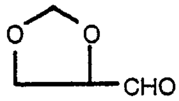
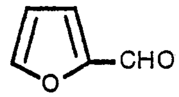
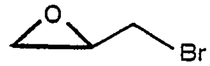

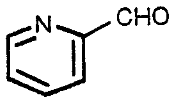
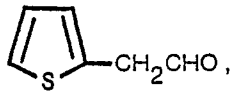
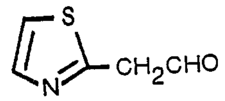

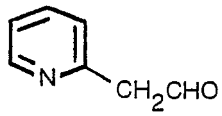
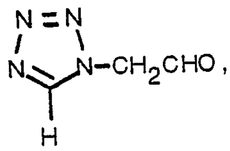
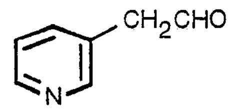

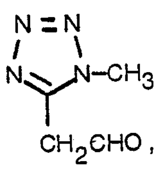
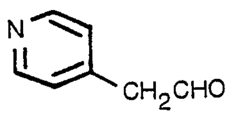
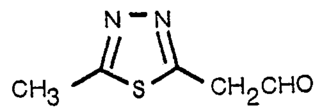

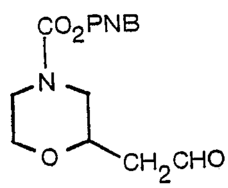
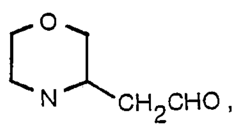
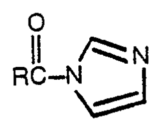 ;

R = $CH_3$, $ClCH_2$, $CH_3CH_2$, $N_3CH_2$, $CH_3OCH_2$, 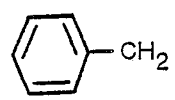

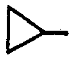 ,
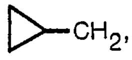
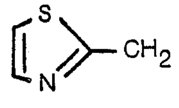

and wherein the alkyl moieties associated with said heterocyclic moieties have 1—6 carbon atoms; $R^6$ and $R^7$ are not both hydrogen at the same time; when $R^6$/$R^7$ is hydrogen, $R^7$/$R^6$ is not

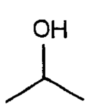

or

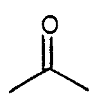

The compounds of the invention are intermediates useful for the preparation of certain 1-carbapenems and their pharmaceutically acceptable salt, ester and amide derivatives which are useful as antibiotics. Such compounds may generally by represented by the following structural formula:

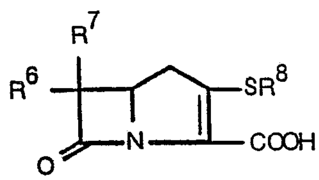

wherein $R^6$, $R^7$, and $R^8$ are independently selected from the group consisting of hydrogen, substituted and unsubstituted: alkyl, alkenyl, and alkynyl, having from 1—10 carbon atoms; cycloalkyl, cycloalkylalkyl, and alkylcycloalkyl, having 3—6 carbon atoms in the cycloalkyl ring and 1—6 carbon atoms in the alkyl moieties; phenyl; aralkyl, aralkenyl, and aralkynyl wherein the aryl moiety is phenyl and the aliphatic portion has 1—6 carbon atoms; the above-named heteroaryl, heteroaralkyl, heterocyclyl and heterocyclylalkyl; wherein the substituent or substituents relative to the above-named radicals are selected from:

$-X°$ halo (chloro, bromo, fluoro)

$-OH$ hydroxy

$-OR^1$ alkoxy, aryloxy

$-O\overset{O}{\overset{\|}{C}}NR^1R^2$ carbamoyloxy

$-\overset{O}{\overset{\|}{C}}NR^1R^2$ carbamoyl

$-NR^1R^2$ amino

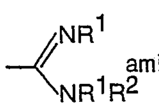 amidino

$-SO_2NR^1R^2$ sulfonamido

$-NH\overset{O}{\overset{\|}{C}}NR^1R^2$ ureido

$R^1\overset{O}{\overset{\|}{C}}NR^2-$ amido

$-CO_2H$ carboxy

$-CO_2R^1$ carboxylate

$-\overset{O}{\overset{\|}{C}}R^1$ acyl

$-O\overset{O}{\overset{\|}{C}}R^1$ acyloxy

$-SH$ mercapto

$-\overset{O}{\overset{\|}{S}}R^1$ alkyl and aryl sulfinyl

$-\overset{O}{\overset{\|}{\underset{O}{\underset{\|}{S}}}}R^1$ alkyl and aryl sulfonyl

$-CN$ cyano

$-N_3$ azido

wherein, relative to the above listed substituents on $R^6$, $R^7$, and $R^8$, the groups $R^1$ and $R^2$ are independently selected from: hydrogen, alkyl, alkenyl, and alkynyl, having from 1—10 carbon atoms; cycloalkyl, cycloalkylalkyl, and alkylcycloalkyl, having 3—6 carbon atoms in the cycloalkyl ring and 1—6 carbon atoms in the alkyl moieties; phenyl; aralkyl, aralkenyl, and aralkynyl wherein the aryl moiety is phenyl and the aliphatic portion has 1—6 carbon atoms; the above-named heteroaryl, heteroaralkyl, heterocyclyl and heterocyclylalkyl and wherein the alkyl moieties associated with said heterocyclic moieties have 1—6 carbon atoms.

Further end products which may be prepared using the compounds of the invention as intermediates are represented by the following generic structure (I):

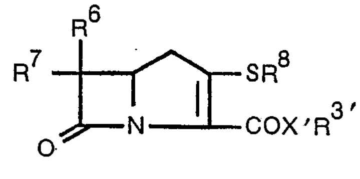

I

wherein X' is oxygen, sulphur or NR' (R' = H or lower alkyl having 1—6 carbon atoms); and $R^{3'}$ is, *inter alia*, representatively selected from the group consisting of hydrogen, conventional blocking groups such as trialkylsilyl, acyl and the pharmaceutically acceptable salt, ester and amide moieties known in the bicyclic β-lactam antibiotic art; the definition of $R^{3'}$ is given in greater detail below.

The total synthesis of the above-named antibiotics using the intermediates of the invention and starting from an appropriately substituted 4-allylazetidinone (II) proceeds *via* intermediates III and IV:

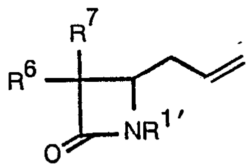

II

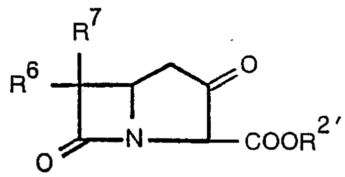

III

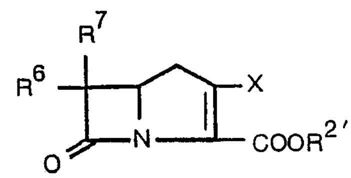

IV

wherein $R^6$ and $R^7$ are as previously defined; X is a conventional leaving group and $R^{2'}$ is hydrogen, a pharmaceutically acceptable ester moiety or a conventional, readily removable protecting group or salt cation. For intermediates III, $R^{2'}$ is as defined but preferably is an ester moiety defined under $R^{2'}$. $R^{1'}$ is hydrogen or a readily removable protecting group such as a triorganosilyl group.

The details of the total synthesis are given below.

The final compounds prepared from the intermediates of the invention are disclosed in European patent applications 78 101 157 and 80 102 076.

U.S. Patent 4,347,367 discloses a multistep process for preparing carbapenems (see steps 3—19 in columns 2 and 3). In comparison with this known process, only five steps are required to make the products from the intermediates of the invention.

Thus, it is an object of the present invention to provide a novel class of intermediates for the preparation of antibiotics which are useful in animal and human therapy and in inanimate systems. These antibiotics are active against a broad range of pathogens which representatively include both gram positive bacteria such as S. aureus, Strep. pyogenes, and B. subtilis, and gram negative bacteria such as E. coli, Pseudomonas, Proteus morganii, Serratia and Klebsiella.

The total synthesis of the compounds as previously defined may conveniently be summarized by the following reaction diagram:

DIAGRAM I

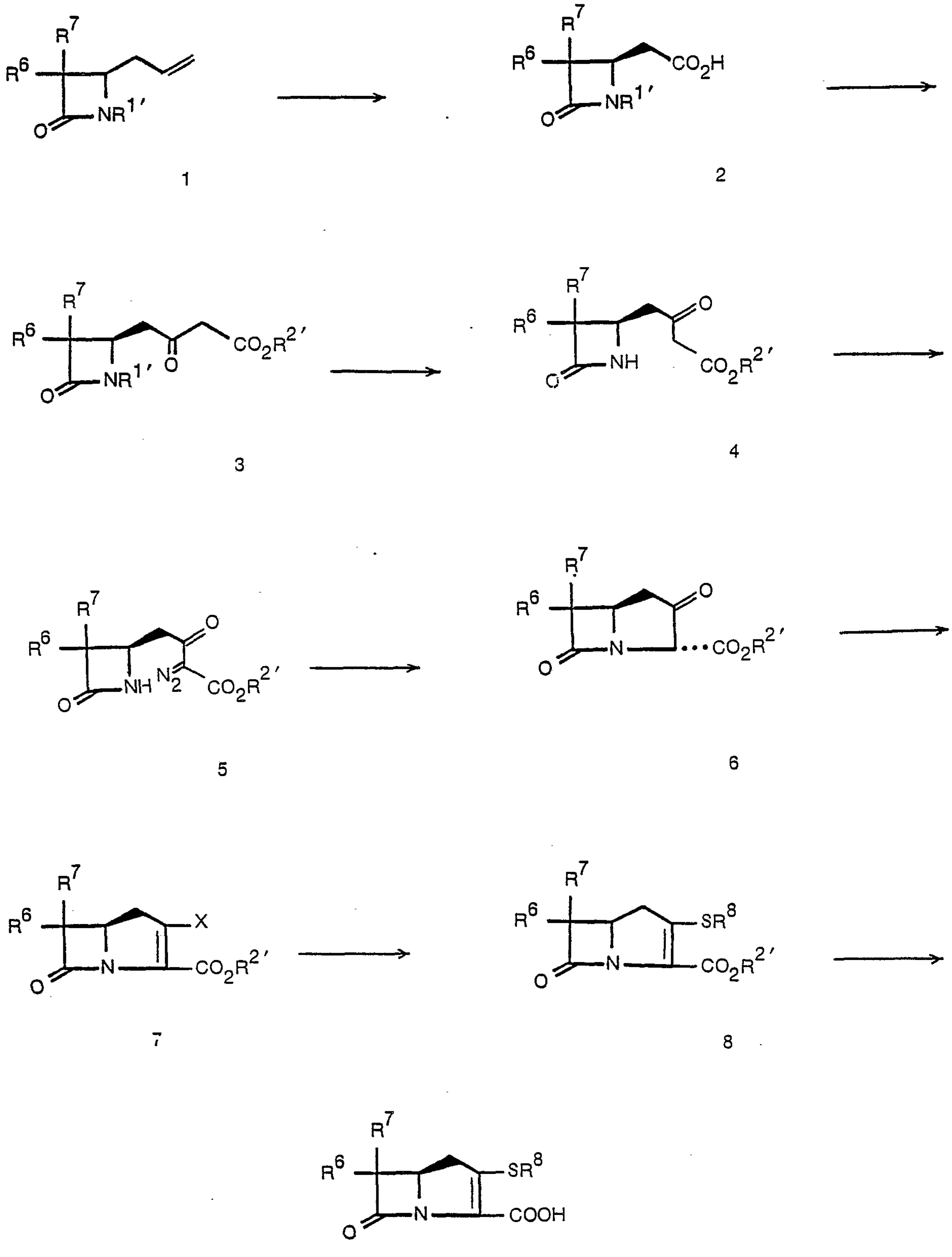

1

2

3

4

5

6

7

8

I

In words relative to Diagram I, oxidation of starting material 1 (described below) is accomplished by treating 1 in a solvent such as methylene chloride, methanol, chloroform or dichloroethane with an oxidizing agent such as ozone at a temperature of from −100 to 0°C for from 0.1 to 4 hours, followed by treating the crude product with an oxidizing agent such as m-chloroperbenzoic acid, hydrogen peroxide, peracetic acid at a temperature of from 0 to 100°C for from 1 to 100 hours to form 2. Chirality is conveniently introduced at this stage of the synthesis. Resolution of racemic 2 can be accomplished, for example, by fractional crystallization of the carboxylate salt formed with an optically active base such as brucine, N-methylphenethylamine or N,N-dimethylphenethylamine.

The addition 2→3 is accomplished by treating 2 with 1,1'-carbonyldimidazole in a solvent such as tetrahydrofuran, dimethoxyethane or DMF at a temperature of from 0 to 50°C, followed by the addition of 0.5 to 3.0 equivalents of $(R^{2'}O_2CCH_2CO_2)_2Mg$, at a temperature of from 0 to 50°C for from 1 to 48 hours. $R^{2'}$ is a readily removable carboxyl protecting group such as p-nitrobenzyl or benzyl. It should also be noted that $R^{2'}$ may be a pharmaceutically acceptable ester moiety; such ester groups are representatively mentioned below. (DMF is dimethylformamide.)

Removal of protecting group $R^{1'}$(3→4) may be accomplished by a variety of known procedures such as hydrolysis or hydrogenation. When $R^{1'}$ is a triorganosilyl group (for example, $[(CH_3)_3C](CH_3)_2Si$—) removal is typically accomplished by acidic aqueous hydrolysis of 3 in a solvent such as methanol, ethanol, tetrahydrofuran, dioxane or DMF in the presence of an acid such as hydrochloric, sulfuric or acetic acid at a temperature of from 0 to 100°C for from 2 to 18 hours.

It should be noted that an otherwise identical deblocking can occur on 1 or 2. Thus, when $R^{1'}$=H, the chain elongation can proceed directly from 2 to 4.

The diazo species 5 is prepared from 4 by treating 4 in a solvent such as $CH_3CN$, $CH_2Cl_2$ or THF with an azide such as p-carboxybenzenesulfonylazide, toluenesulfonylazide or methanesulfonylazide in the presence of a base such as triethylamine, pyridine or $(C_2H_5)_2NH$, for from 1 to 50 hours at 0—25°C. (THF is tetrahydrofuran.)

Cyclization (5→6) is accomplished by treating 5 in a solvent such as benzene, toluene or THF at a temperature of from 50—110°C for from 1—5 hours in the presence of a catalyst such as *bis* (acetylacetonato)Cu(II) $[Cu(acac)_2]$, $CuSO_4$, Cu powder, $Rh(OAc)_2$, or $Pd(OAc)_2$. Alternatively, the cyclization may be accomplished by irradiating 6 through a pyrex filter (a wave length greater than 300 nm) in a solvent such as benzene, $CCl_4$ or diethylether at a temperature of from 0—25°C for from 0.5 to 2 hours. ["OAc" = acetate.]

Establishment of leaving group X (6→7) is accomplished by acylating the keto ester 6 with an acylating agent R°X such as p-toluenesulfonic acid anhydride, p-nitrophenylsulfonic acid anhydride, 2,4,6-triisopropylphenylsulfonic acid anhydride, methanesulfonic acid anhydride, trifluoromethane sulfonic acid anhydride, diphenyl chlorophosphate, toluenesulfonyl chloride, p-bromophenylsulfonyl chloride; wherein X is the corresponding leaving group such as toluene sulfonyloxy, p-nitrophenylsulfonyloxy, diphenylphosphoryl, and other leaving groups which are established by conventional procedures and are well known in the art. Typically, the above acylation to establish leaving groups X is conducted in a solvent such as methylene chloride, acetonitrile or dimethylformamide, in the presence of a base such as diisopropylethylamine, triethylamine, 4-dimethylamino-pyridine at a temperature of from −20 to 40°C for from 0.1 to 5 hours. The leaving group X of intermediate 7 can also be halogen. The halogen leaving group is established by treating 7 with a halogenating agent such as $Ø_3PCl_2$, $Ø_3PBr_2$, $(ØO)_3PBr_2$, oxalyl chloride in a solvent such as $CH_2Cl_2$, $CH_3CN$, THF, in the presence of a base such as diisopropylethylamine, triethylamine, or 4-dimethylaminopyridine. [Ø = phenyl]

The reaction 7→8 is accomplished by treating 7 in a solvent such as dioxane, dimethylformamide, dimethylsulfoxide, acetonitrile, hexamethylphosphoramide, in the presence of an approximately equivalent to excess of the mercaptan reagent $HSR^8$, wherein $R^8$ is as defined above, in the presence of a base such as sodium hydrogen carbonate, potassium carbonate, triethylamine, diisopropylethylamine, at a temperature of from −40 to 25°C for from 1 to 72 hours. When $R^8$ is substituted by a primary or secondary amino group, for example $—CH_2CH_2NH_2$, the mercaptan reagent may be represented as $HSCH_2CH_2NHR°$, for example; wherein R° is a readily removable N-protecting group such as p-nitrobenzyloxycarbonyl, $(—CO_2pNB)$, o-nitrobenzyloxycarbonyl. The specifically illustrated mercaptan reagent, $HSCH_2CH_2NHR°$, is typically prepared by treating aminoethylmercaptan in the presence of the desired acid chloride in the presence of a base such as sodium bicarbonate, sodium hydroxide, in a solvent such as aqueous diethylether, aqueous dioxane, aqueous acetone, at a temperature of from 0 to 25°C for from 0.5 to 4 hours. The foregoing mercaptan reagent, $HSR^8$, and means for its protection, is simply illustrative. The class of suitable $HSR^8$ reagents is representatively described below and in the Examples.

The final deblocking step 8→I is accomplished by conventional procedures such as solvolysis or hydrogenation. Typically 8 in a solvent such as dioxane-water-ethanol, tetrahydrofuran-aqueous dipotassium hydrogen phosphate-isopropanol is treated under a hydrogen pressure of from 1 to 4 atmospheres in the presence of a hydrogenation catalyst such as palladium on charcoal, palladium hydroxide, platinum oxide, at a temperature of from 0 to 50°C for from 0.25 to 4 hours to provide I. Photolysis, when $R^2$ is a group such as o-nitrobenzyl, for example, may also be used for deblocking.

Introduction of the Thia Side Chain

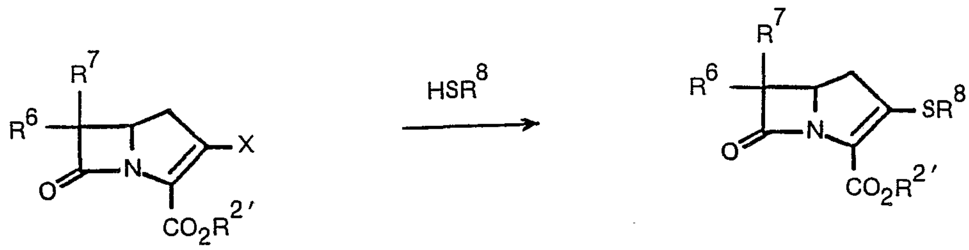


7 8

Suitable reagents $HSR^8$ utilized in the transformation 7→8 are listed below. The list is arranged according to structural and functional characteristics of the thia side chain $—SR^8$; annotation is provided where necessary. The thia side chain of choice is derived from the corresponding mercaptan reagent $HSR^8$. When the mercaptan contains a functional group which might interfere with the intended course of reaction, the offending group is covered. For example, when a basic nitrogen group is encountered (—NHR or $—NH_2$, for example) it is usually protected by acylation (e.g., $—CO_2pNB$) and when a carboxyl group ($—CO_2H$) is present, it is usually protected by esterification (e.g., PNB ester). Such protection also facilitates in the purification of products 8 by chromatographic means. (pNB is p-nitrobenzyl).

1.) *Aliphatic (including carbocyclic) Mercaptans:* $HSR^8$ wherein $R^8$ is 1—10 carbon alkyl, cycloalkyl, alkenyl, cycloalkenyl, or alkynyl; $R^8$ may be branched or unbranched.

EXAMPLES

$HSCH_3$

$HSCH_2CH_3$

$HSCH_2CH_2CH_3$

$HSCH(CH_3)_2$

$HS(CH_2)_3CH_3$

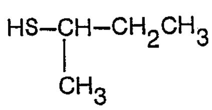

$HSCH_2CH(CH_3)_2$

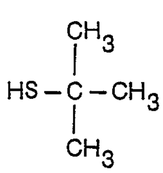

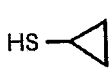

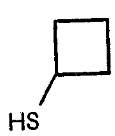

EXAMPLES (Continued)

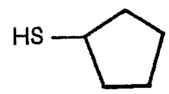

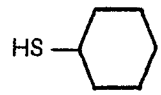

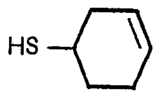

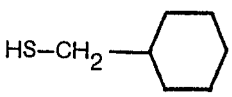

$HS{-}CH_2{-}CH{=}CH_2$

$HS{-}CH_2{-}CH{=}C(CH_3)_2$

$HS{-}CH_2{-}C{\equiv}CH$

$HS{-}CH_2{-}C{\equiv}C{-}CH_3$

2.) *Substituted Aliphatic Mercaptans:* $HSR^8$ wherein $R^8$ is a 1—10 carbon branched or unbranched alkyl, cycloalkyl, alkenyl, cycloalkenyl or alkynyl group substituted by one or more halo,

$OH$, $OR^1$, $O\overset{O}{\overset{\|}{C}}R^1$, $O\overset{O}{\overset{\|}{C}}NH_2$, $O\overset{O}{\overset{\|}{C}}NR^1R^2$, $NH_2$, $NHR^1$, $NR^1R^2$, $\overset{O}{\overset{\|}{C}}R^1$, $CO_2H$, $CO_2R^1$,

$CONH_2$, $CONHR^1$, $CONR^1R^2$, $CN$, $SR^1$, $\overset{O}{\overset{\|}{S}}R^1$, $SO_2R^1$, $SO_2NH_2$, $SO_2NHR^1$,

$SO_2NR^1R^2$, $NH\overset{O}{\overset{\|}{C}}R^1$, $NH\overset{O}{\overset{\|}{C}}NH_2$, $NH\overset{O}{\overset{\|}{C}}NHR^1$, $NH\overset{O}{\overset{\|}{C}}NR^1R^2$, $NH\overset{O}{\overset{\|}{C}}OR^1$, $\overset{NH}{\overset{\|}{C}}NH_2$, $\overset{NR^1}{\overset{\|}{C}}NHR^2$,

wherein $R^1$ and $R^2$ are as previously defined relative to substituents on $R^8$. Preferred substituents are basic nitrogen containing groups.

EXAMPLES

$HS(CH_2)_nOR^1$ $n = 2–4$, $R^1 = H$, $\overset{O}{\overset{\|}{C}}CH_3$, $CH_3$

$HS(CH_2)_n\overset{O}{\overset{\|}{C}}XR$ $n = 1–3$, $X = O$, $NH$, $NR^1$, $R^1 = H$, $CH_3$

$HS(CH_2)_nNH_2$ $n = 2–4$

$HS(CH_2)_nNHR^1$ $n = 2–4$, $R^1 = CH_3$, $CH_2CH_3$, $CH_2CH_2CH_3$, $\overset{O}{\overset{\|}{C}}CH_3$

$HS(CH_2)_nNR^1R^2$ $n = 2–4$, $R^1/R^2 = CH_3$, $CH_2CH_3$

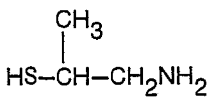

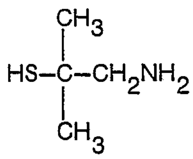

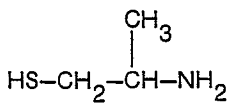

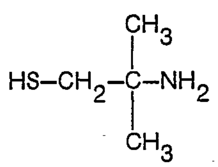

$HS–CH_2CH_2SCH_3$

$HS–CH_2CH_2NHC(CH_3)_3$

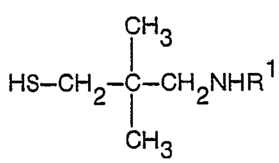 $R^1 = H$, $CH_3$, $\overset{O}{\overset{\|}{C}}CH_3$

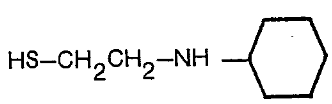

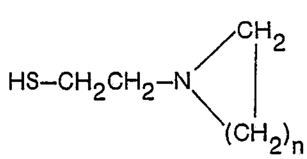 $n = 3–5$

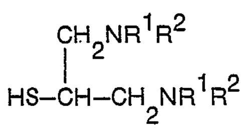 $R^1 = H$, $CH_3$; $R^2 = H$, $CH_3$

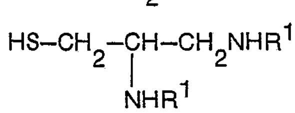 $R^1 = H$, $CH_3$

EXAMPLES (Continued)

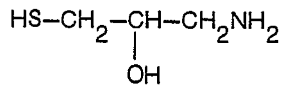

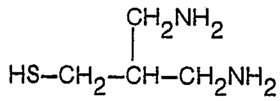

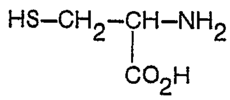

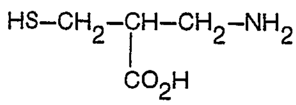

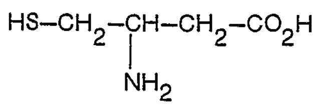

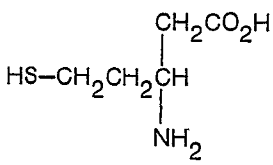

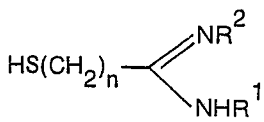

$n = 1–3$, $R^2 = H, CH_3$, $R^1 = H, CH_3$

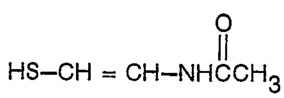

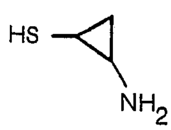

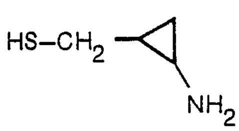

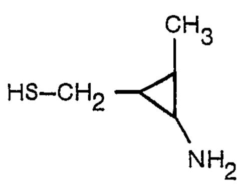

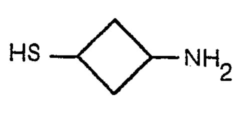

EXAMPLES (Continued)

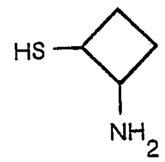

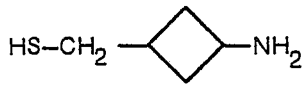

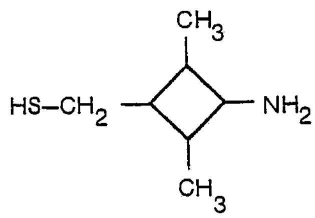

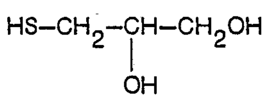

5-thio-D-glucose

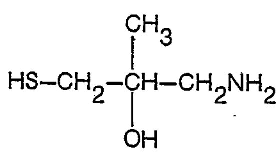

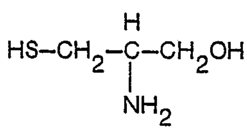

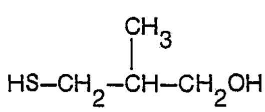

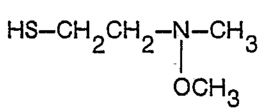

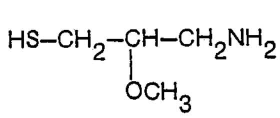

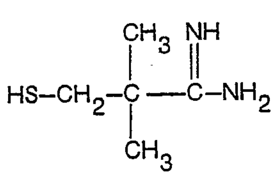

EXAMPLES (Continued)

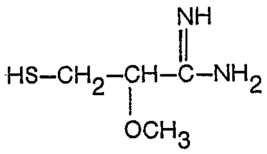

$HS-CH_2-CH = CH-CH_2NH_2$

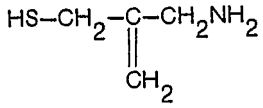

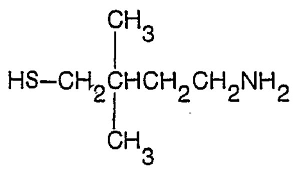

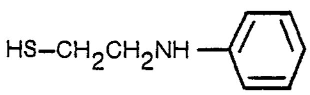

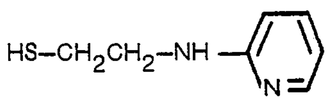

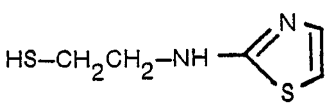

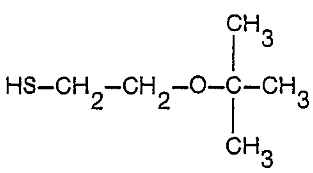

3.) *Aryl Mercaptans:* $HSR^8$ wherein $R^8$ is phenyl or substituted phenyl. The substituents are independently selected from those previously defined for $R^8$. Especially preferred substituents include alkyl, halo, hydroxy, alkoxy, acyloxy, acyl, carboxy, mercapto, sulfinyl, sulfonyl, amino, substituted amino, aminoalkyl, substituted aminoalkyl, amide, and ureido.

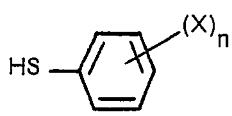 n=1, 2 or 3, X=f, Cl, Br, OH, OR, $O\overset{O}{\overset{\|}{C}}R^1$, $NH_2$, $NHR^1$, $NR^1R^2$, $CH_2NH_2$, $CH_2NR^1R^2$,

$CO_2H$, $CO_2R^1$, $COR^1$, $CONH_2$, $CONR^1R^2$, $R^1CONH$, $R^1NHCONH$, $SR^1$, $\overset{O}{\overset{\|}{S}}R^1$, $SO_2R^1$, $CH_3$, $CF_3$; $R^1$ and $R^2$ are as previously defined under $R^8$.

EXAMPLES

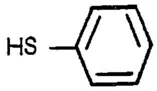

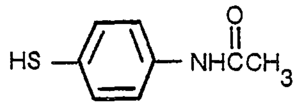

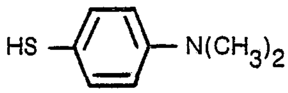

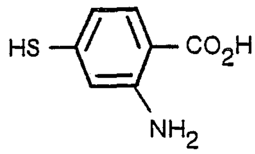

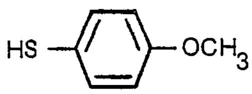

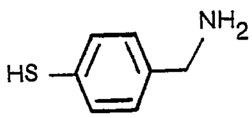

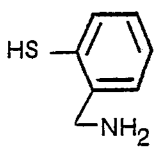

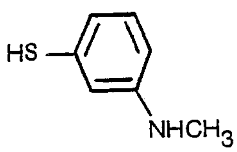

4.) *Heteroaryl Mercaptans:* $HSR^8$ wherein $R^8$ is a substituted or unsubstituted heteroaryl group containing 1—4 O, N or S atoms. Typical substituents include those mentioned above under "Aryl Mercaptans".

EXAMPLES

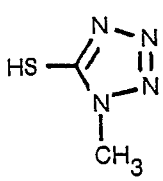

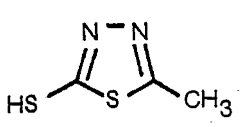

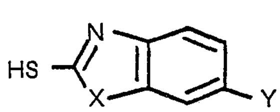

X = N, O  Y = H

X = S  Y = H, Cl, $OCH_2CH_3$

EXAMPLES (Continued)

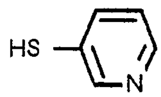

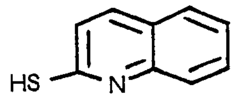

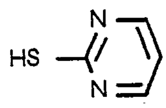

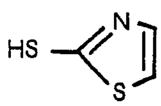

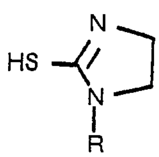

R = H, $CH_3$

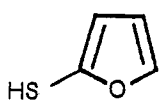

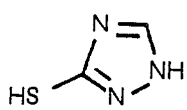

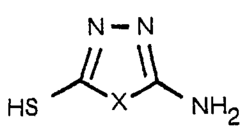

X = NH, S

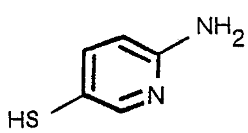

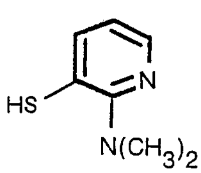

5.) *Arylaliphatic Mercaptans:* $HSR^8$ where $R^8$ is a 1—6 carbon branched or unbranched alkyl, cycloalkyl, alkenyl, or alkynyl group substituted by a phenyl or substituted phenyl group. Typical phenyl substituents include those mentioned under "Aryl Mercaptans".

EXAMPLES

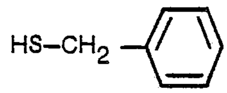

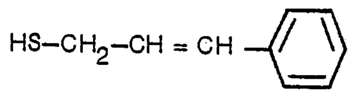

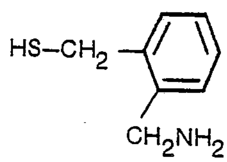

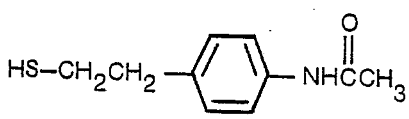

6.) *Heteroarylaliphatic and Heterocyclicaliphatic Mercaptans* $HSR^8$ wherein $R^8$ is a 1—6 carbon branched or unbranched alkyl, cycloalkyl, alkenyl, or alkynyl group substituted by a heteroaryl or heterocyclic group containing 1—4, O, N, or S atoms. The heteroaryl or heterocyclic group is unsubstituted or substituted by those substituents mentioned under "Aryl Mercaptans", (No. 3, above).

EXAMPLES

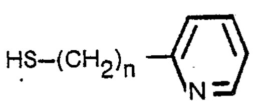

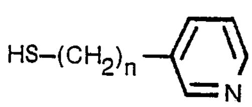

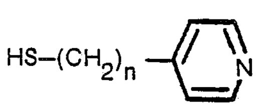

$n = 1,2$

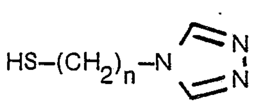

EXAMPLES (Continued)

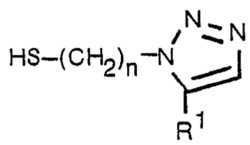

$R^1 = OCH_2CH_3$

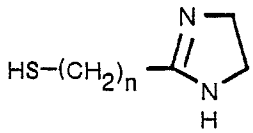

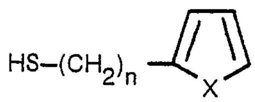

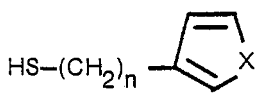

X = O, S, NH

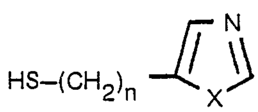

X = O, S, NH

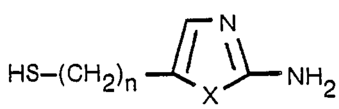

X = O, S, NH

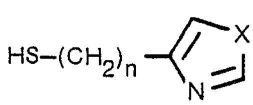

X = O, S, NH

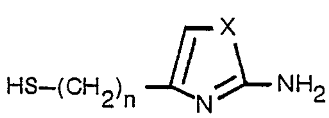

X = O, S, NH

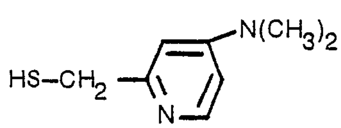

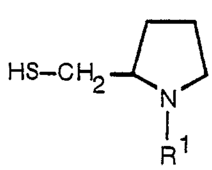

$R^1$ = H, $CH_3$

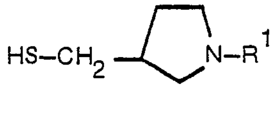

$R^1$ = H, $CH_3$

EXAMPLES (Continued)

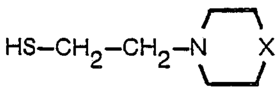

$X = O, NH, NCH_3$

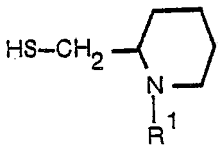

$R^1 = H, CH_3$

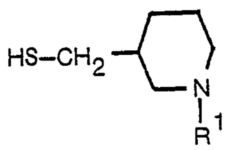

$R^1 = H, CH_3$

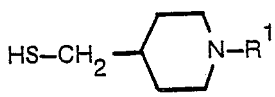

$R^1 = H, CH_3$

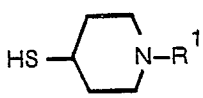

$R^1 = H, CH_3$

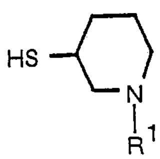

$R^1 = H, CH_3$

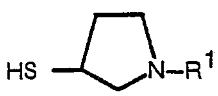

$R^1 = H, CH_3$

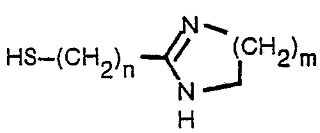

$n = 1–3$, $m = 1–3$

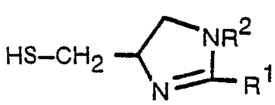

$R^2 = H, CH_3$, $R^1 = H, CH_3, NH_2$

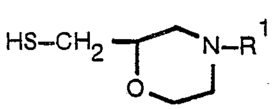

$R^1 = H, CH_3$

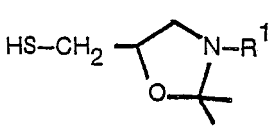

$R^1 = H, CH_3$

7.) *Alkyl-Heteroatom-Alkyl Mercaptans, $HSR^8$*
Wherein $R^8$ is

$$—(CH_2)_nX(CH_2)_mR^9$$

wherein n = 2 to 4, m = 2 to 4; X is NR°, O or S;
and wherein R° is H, $CH_3$, $CH_2CH_3$, $CH_2CH_2OH$, or $CH_2CH_2NH_2$ and $R^9$ is OH, $NH_2$, $NHCH_3$, $N(CH_3)_2$,

$$O\underset{\underset{\displaystyle O}{\|}}{C}CH_3, \qquad NH\underset{\underset{\displaystyle O}{\|}}{C}CH_3.$$

Note, in the above representation, the methylene carbons may be branched; for example:

$$\underset{\displaystyle CH_3}{\underset{|}{—CH—}}, \qquad \overset{\displaystyle CH_3}{\overset{|}{\underset{\displaystyle CH_3}{\underset{|}{—C—}}}}.$$

The following $HSR^8$ are representative of this class:

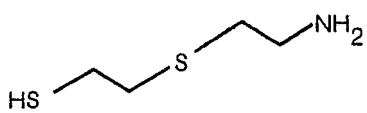

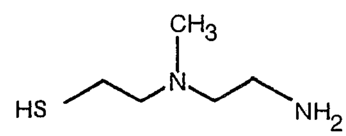

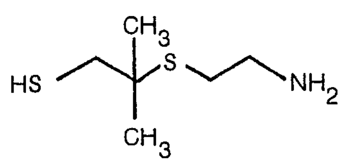

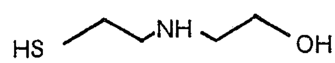

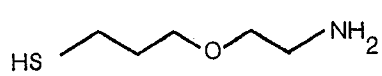

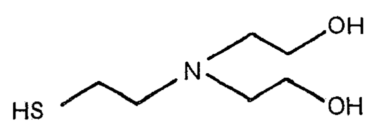

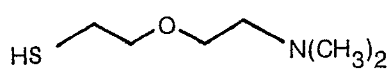

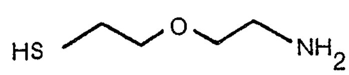

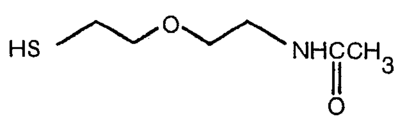

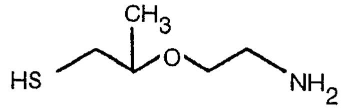

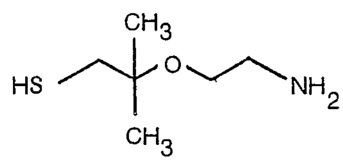

PREPARATION OF THE SUBSTITUTED 4-ALLYLAZETIDINONE, 1

Starting material 1 is conveniently prepared from 4-allylazetidinone according to the following scheme:

SCHEME I

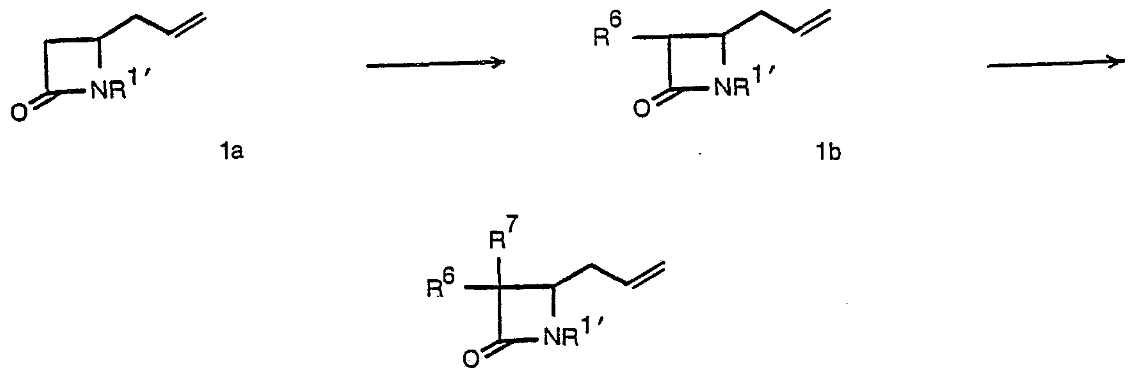

1a

1b

1

It should be noted that starting material 1a is known. $R^{1'}$ is a removable blocking group such as triorganosilyl, for example, trimethylsilyl, t-butyldimethylsilyl or triphenylsilyl.

In words relative to the above reaction diagram starting material 1a can be mono-, or dialkylated at ring position 3. Alkylation of 1a provides 1b. Typically, 1a is treated with a strong base such as lithium diisopropylamide, lithium 2,2,6,6-tetramethylpiperidide, potassium hydride, lithium hexamethyldisilazane, phenyllithium in a solvent such as tetrahydrofuran (THF), hexamethylphosphoramide, ether, dimethoxyethane, at a temperature of from −80 to 0°C whereupon the alkylating agent of choice, $R^6X^\circ$ is added ($X^\circ$ is chloro, iodo or bromo); alternatively the alkylating agent may be $R^6$-tosylate, $R^6$-mesylate or an aldehyde or ketone such as acetaldehyde to provide monoalkylated species 1b. When desired, dialkylated species 1 may be obtained from 1b by repeating the alkylating procedures 1a →1b. The 6-substituents can also be established by direct acylation using an acylating agent such as N-acyl imidazole. Such N-acyl imidazole acylating reagents are listed below. Also given below is a detailed description of this second approach for establishing, $R^6$ and $R^7$. The following list is representative of useful alkylating agents for establishing $R^6$ and $R^7$, according to the above scheme: 1a→1b→1:

ALKYLATING AGENTS

$CH_3CHO$

$\phi CH_2CHO$ $\phi$ = phenyl

$\phi CH_2CH_2CHO$

$CH_2O$

$CH_3I$

$\phi CH_2Br$

$CH_3COCH_3$

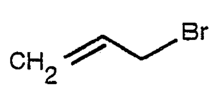

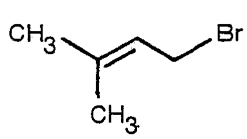

ALKYLATING AGENTS (Continued)

$CH_3OCH_2CHO$

$CH_3CH_2I$

$(CH_3)_2CHI$

$N_3CH_2CHO$

$Me_2NCH_2CHO$

$RO_2CCH_2Br$ R = $CH_3$, benzyl, p-nitrobenzyl

$CF_3CF_2CHO$

$RO_2CCH_2CHO$ R = $CH_3$, benzyl, p-nitrobenzyl

$CH_3CH(CH_3)CHO$

$CH_3(CH_3)CHCH_2CHO$

$CH_3CH_2CHO$

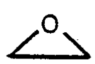

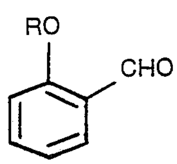

$CF_3CHO$

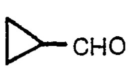

R = protecting group

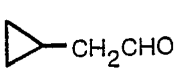

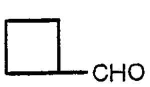

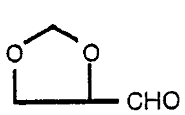

ALKYLATING AGENTS (Continued)

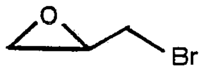

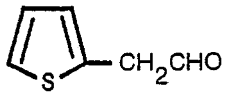

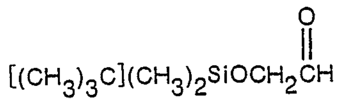

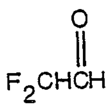

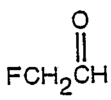

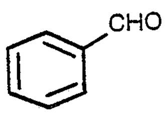

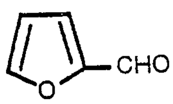

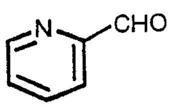

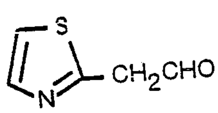

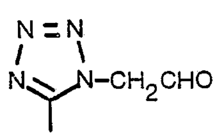

ALKYLATING AGENTS (Continued)

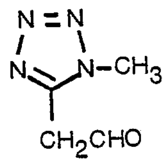

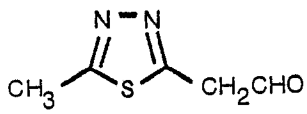

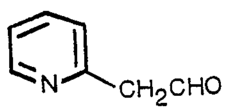

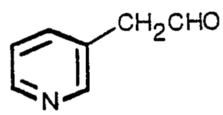

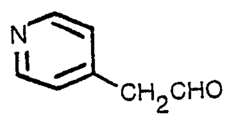

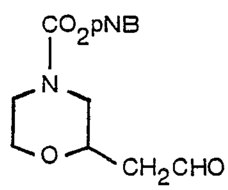

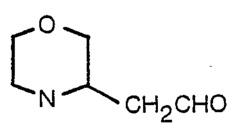

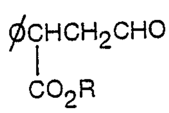

R is removable carboxyl protecting group, such as benzyl.

As mentioned above, the 6-substituents may also be established by acylation. Utilization of such acylating agents may be demonstrated in the following manner with regard to a preferred starting material 1:

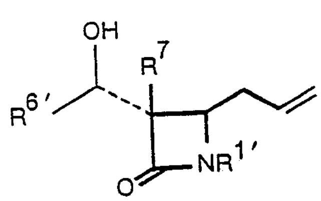

1

wherein $R^7$ and $R^{1'}$ are as defined above. $R^{6'}$ is defined relative to the definition of $R^6$ and in that sense is the balance of the previously identified group $R^6$. In other words, for purposes of this definition

$R^{6'}CH(OH)- = R^6$. An especially preferred material 1 is when $R^7$ is hydrogen and $R^{6'}$ is methyl. Basically, such 1′-hydroxy $R^{6'}$ species 1 are prepared according to the following scheme:

SCHEME II

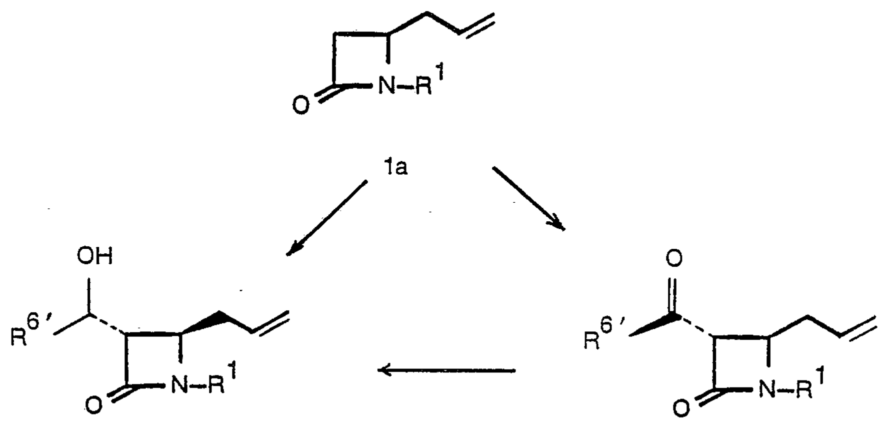

1a

1

1′

The alkylation 1a→1, Scheme II, is accomplished as previously described, by treating 1a in a solvent such as tetrahydrofuran, dimethoxyethane, diethylether, hexamethylphosphoramide, at a temperature of from −100 to −20°C with a strong base such as lithium diisopropylamide, lithium hexamethyldisilazide, lithium 2,2,6,6-tetramethylpiperidide, potassium hydride followed by the addition of an equivalent to 10 fold excess of an aldehyde. This reaction gives a mixture of isomers from which the desired *trans*-R form 1 can be conveniently separated by chromatography or crystallization.

Intermediate 1a may proceed directly to 1 as indicated above, or it may take the circuitous path *via* 1′. The direct acylation, to 1′ is accomplished by treating 1a with two or more equivalents of a base such as lithium diisopropylamide, lithium hexamethyldisilazide, lithium 2,2,6,6-tetramethylpiperidide, in a solvent such as tetrahydrofuran, diethylether, or dimethoxyethane, for example, at a temperature of from −100 to −20°C with an acylating agent such as N-acyl imidazole. Addition of the 1a plus base mixture to the acylating agent is preferred.

Representative acylating agents for this scheme 1a→1′→1 are listed below.

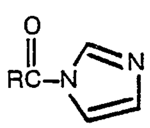 ; R = $CH_3$, $ClCH_2$, $CH_3CH_2$, $N_3CH_2$, $CH_3OCH_2$,

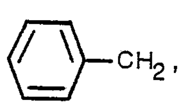, 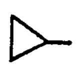, 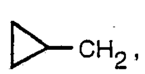, 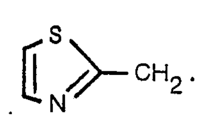.

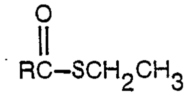 ; R = $CF_3$, $CF_2H$, $CH_2=CH$, 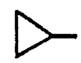,

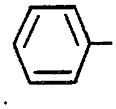, 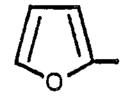.

Further with respect to Scheme II, the reduction, 1'→1 is accomplished by contacting the ketone with a reducing agent such as potassium tri(sec-butyl)borohydride, lithium tri(sec-butyl)borohydride, sodium borohydride, sodium tris(methoxyethoxy)aluminum hydride, lithium aluminum hydride in a solvent such as diethylether, tetrahydrofuran, toluene at a temperature of from −78 to 25°C. The reaction can conveniently be conducted in the presence of an added complexing salt such as potassium iodide or magnesium bromide.

In a similar manner, unresolved 1 (*cis* and *trans*) may be oxidized to 1' for reduction to 1 as indicated above:

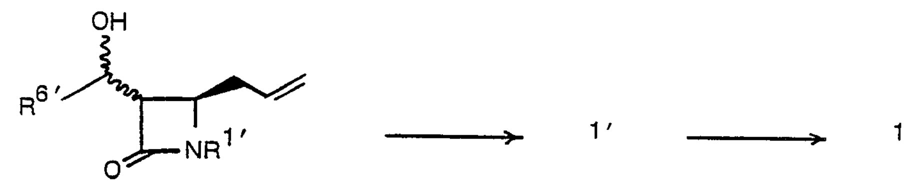


The oxidation is accomplished with an oxidizing agent such as dipyridine chromium (VI) oxide, trifluoroacetic anhydride-dimethylsulfoxide-triethylamine, pyridinium dichromate, acetic anhydride-dimethylsulfoxide in a solvent such as methylene chloride or acetonitrile at a temperature of from −78 to 25°C for from 5 minutes to 5 hours.

As noted above, the final products derived from the intermediates of the invention may also generally be represented by the following structural formula:

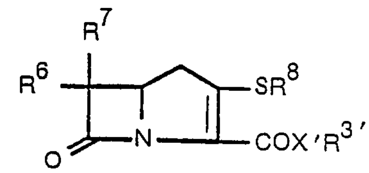

I

wherein X' is oxygen, sulfur or NR' (R' is hydrogen or loweralkyl having from 1 to 6 carbon atoms); and $R^{3'}$ is hydrogen, or, *inter alia*, is representatively selected to provide the pharmaceutically acceptable salt, ester anhydride ($R^{3'}$ is acyl) and amide moieties known in the bicyclic β-lactam antibiotic art; $R^{3'}$ may also be a readily removable blocking group.

Identification of the Radical —COX'$R^{3'}$

In the generic representation of the compounds (I, above), the radical represented by —COX'$R^{3'}$ is, *inter alia*, —COOH (X' is oxygen and $R^{3'}$ is hydrogen) and all radicals known to be effective as pharmaceutically acceptable ester, anhydride ($R^{3'}$ is acyl) and amide radicals in the bicyclic β-lactam antibiotic art, such as the cephalosporins and penicillins and nuclear analogues thereof.

Suitable blocking esters ($R^{3'}$, X' = O) include those selected from the following list which is representative:

(i) $R^{3'}$ = $CR^aR^bR^c$ wherein at least one of $R^a$, $R^b$ and $R^c$ is an electron-donor, e.g., *p*-methoxyphenyl. The remaining $R^a$, $R^b$ and $R^c$ groups may be hydrogen or organic substituting groups. Suitable ester groups of this type include *p*-methoxybenzyloxycarbonyl.

(ii) $R^{3'}$ = $CR^aR^bR^c$ wherein at least one of $R^a$, $R^b$ and $R^c$ is an electron-attracting group, e.g., *p*-nitrophenyl, trichloromethyl, and o-nitrophenyl. Suitable esters of this type include *p*-nitrobenzyloxycarbonyl, and 2,2,2-trichloroethoxycarbonyl.

(iii) $R^{3'}$ = $CR^aR^bR^c$ wherein at least two of $R^a$, $R^b$ and $R^c$ are hydrocarbon such as alkyl, e.g., methyl or ethyl, or aryl, e.g., phenyl and the remaining $R^a$, $R^b$ and $R^c$ group, if there is one, is hydrogen. Suitable esters of this type include t-butyloxycarbonyl, diphenylmethoxycarbonyl and triphenylmethoxycarbonyl.

Silyl esters, under this category of blocking groups, may conveniently be prepared from a halosilane of the formula:

$$R^4_3SiX'$$

wherein X' is a halogen such as chloro or bromo and $R^4$ is alkyl, e.g., methyl, ethyl, t-butyl.

Pharmaceutically acceptable carboxyl derivatives are those derived by reacting I with alcohols or acylating reagents.

For example, esters and amides of interest are the above-listed starting materials and final products having the —COX'$R^{3'}$ group at the 3-position; wherein X' is oxygen, sulfur or NR' (R' is H or $R^{3'}$), and $R^{3'}$ is alkyl having 1—6 carbon atoms, straight or branched, such as methyl, ethyl or t-butyl; carbonylmethyl, including phenacyl; aminoalkyl including 2-methylaminoethyl, 2-diethylaminoethyl; alkanoyloxyalkyl wherein the alkanoyloxy portion is straight or branched and has 1—6 carbon atoms and the alkyl portion has 1—6 carbon atoms, such as pivaloyloxymethyl; haloalkyl wherein halo is chloro, and the alkyl portion is straight or branched having 1—6 carbon atoms, e.g., 2,2,2-trichloroethyl; alkenyl having 1—4 carbon atoms such, as 2-propenyl, 3-butenyl, and 4-butenyl; aralkyl and lower alkoxyl- and nitro- substituted aralkyl such as benzyl, benzhydryl, o-nitrobenzyl, p-methoxybenzyl, and p-nitrobenzyl; phthalidyl; benzyloxyalkyl having 8—10 carbon atoms such as benzyloxymethyl, and (4-nitro) benzyloxymethyl.

In addition to the esters (and thio esters) listed above, amides are also suitable, i.e., wherein X' is the

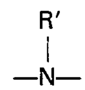

group. Representatives of such amides are those wherein R' is selected from the group consisting of hydrogen and lower alkyl such as methyl and ethyl.

The most preferred —COX'$R^{3'}$ radicals invention are those wherein (relative to Structure I above), X' is oxygen and $R^{3'}$ is hydrogen; loweralkyl having 1—4 carbon atoms; lower alkenyl such as 3-methylbutenyl or 4-butenyl; benzyl and substituted benzyl such as p-nitrobenzyl; pivaloyloxymethyl, 3-phthalidyl; and phenacyl.

The compounds of the formula (I) are valuable antibiotics active against various gram-positive and gram-negative bacteria and accordingly find utility in human and veterinary medicine. Representative pathogens which are sensitive to antibiotics I include: *Staphyloccus aureus, Escherichia coli, Klebsiella pneumoniae, Bacillus subtilis, Salmonella typhosa, Psuedomonas* and *Bacterium proteus*. The antibacterials are not limited to utility as medicaments; they may be used in all manner of industry.

The above-mentioned end products may be used in any of a variety of pharmaceutical preparations. They may be employed in capsule, powder form, in liquid solution, or in suspension. They may be adminsitered by a variety of means; those of principal interest include: orally, topically or parenterally by injection (intravenously or intramuscularly).

In the foregoing word description of the above schematic reaction diagram for the total synthesis of the defined carbapenem antibiotics, it is to be understood that there is considerable latitude in selection of precise reaction parameters. Suggestion of this latitude and its breadth is generally indicated by the enumeration of equivalent solvent systems, temperature ranges, protecting groups, and range of identities of involved reagents. Further, it is to be understood that the presentation of the synthetic scheme as comprising distinct steps in a given sequence is more in the nature of a descriptive convenience than as a necessary requirement; for one will recognize that the mechanically dissected scheme represents a unified scheme of synthesis and that certain steps, in actual practice, are capable of being merged, conducted simultaneously, or effected in a reverse sequence without materially altering the progress of synthesis.

The following examples recite a precise scheme of total synthesis. It is to be understood that the purpose of this recitation is to further illustrate the total synthesis. Temperature is given in °C.

Example 1

Preparation of 1-(t-Butyldimethylsilyl)-4-(prop-2-ene)-azetidin-2-one

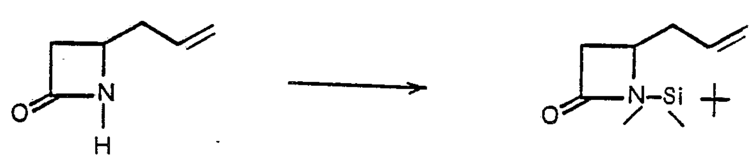

t-Butyldimethylchlorosilane (7.51 g, 49.8 mmol) is added in one portion to an ice-cold, stirring solution of 4-(prop-2-ene)-azetidin-2-one (5.26 g, 47.4 mmol) and triethylamine (5.04 g, 49.8 mml) in anhydrous dimethylformamide (100 ml). A voluminous white precipitate forms almost immediately. The reaction mixture is stirred at 0—5°C for 1 hour and then allowed to warm to room temperature. Most of the solvent is removed under vacuum to give a residue which is partitioned between diethyl ether (250 ml) and water. The ethereal phase is washed with 2.5N hydrochloric acid (50 ml), water (3 × 50 ml), and brine, dried with magnesium sulfate, filtered and evaporated under vacuum to provide an oil which is purified either by

vacuum distillation or chromatography on silica gel (20% ether in petroleum ether) to yield 1-(t-Butyldimethylsilyl-4-(prop-2-end)-azetidin-2-one. n.m.r. ($CnCl_3$) δ 4.8—6.0 (3H, m, olefinic), δ 3.5 (1H, m, H4), δ 3.03 (1H, dd, J=15, 5.2H3x), δ 2.56 (1H, dd, J=15, 2.8, H2β) δ 1.8—2.8 (2H, m, allylic), δ 0.9 (9H, S), δ 0.2 (6H, S).

Example 2

1-(t-Butyldimethylsilyl)-3-(1-hydroxyethyl)-4-(prop-2-ene)-azetidin-2-one

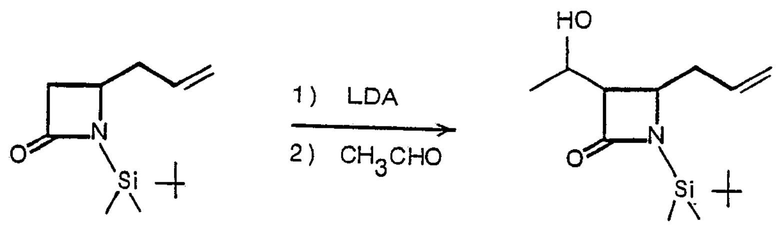


n-Butyllithium in hexane (26.25 mmol) is added slowly by syringe to a solution of diisopropylamine (26.25 mmol) in anhydrous tetrahydrofuran (100 ml) at −78°C. The resulting solution is stirred for 15 min. prior to the addition of a solution of 1-(t-butyldimethylsilyl)-4-(prop-2-ene)-azetidin-2-one (25.0 mmol) in anhydrous tetrahydrofuran (25 ml). After stirring for 15 min at −78°C, acetaldehyde (75 mmol) is added by syringe and the resulting solution is stirred at −78°C for 5 min. Saturated aqueous ammonium chloride solution (15 ml) is added by syringe and the reaction mixture is allowed to warm to room temperature, then diluted with ether (250 ml) and washed with 2.5N hydrochloric acid solution (2 × 50 ml), water (100 ml) and brine and dried over magneisum sulfate. Solvents are removed *in vacuo* and the semi-solid residue is chromatographed on silica gel (1:1, ether:petroleum ether). The first product to elute is the cis *R** compound (688 mg) n.m.r. ($CDCl_3+D_2O$) δ 4.8—6.2 (3H, m, olefinic, δ 4.2 (1H, da, J=6.5, 3.7, H—8), δ 3.75 (1H, ddd, J=5.5, 5, 4.8, H—5), δ 3.28 (H, dd, J=5.5, 3.7, H—6), δ 2.2—3.0 (2H, m, allyl), δ 1.35 (3H, d, J=6.5, $CH_3$—CHOH), δ 1.0 (9H, S, ± Si—), δ 0.3 (6H, S, $(CH_3)_2Si$). The second fraction is a mixture of the trans *R** and *S** products (5.56 g). Crystallization of this material from petroleum ether gives the pure trans *R** material, m.p. 81—82°C. IR ($CHCl_3$) 3400, 2920, 2850, 1723 $cm^{-1}$; n.m.r. ($CDCl_3$ + $D_2O$) δ 4.9—6.2 (3H, m, olefinic), δ 4.1 (1H, dq, J=7.0, 6.8, H8), δ 3.66 (1H, ddd, J=11, 4.5, 3.0, H5), δ 2.9 (1H, dd, J=6.8, 3.0, H6), δ 1.8—2.8 (2H, m, allyl), δ1.26 (3H, d, J=7.0, $CH_3$—), δ 1.0 (9H, S, ± Si), δ 0.28 (6H, 2S, $(CH_3)_2Si$).

Example 3

(3*S**, 4*R**)-1-(t-Butyldimethylsilyl)-3-(1-oxyethyl)-4-(prop-2-ene)-azetidin-2-one

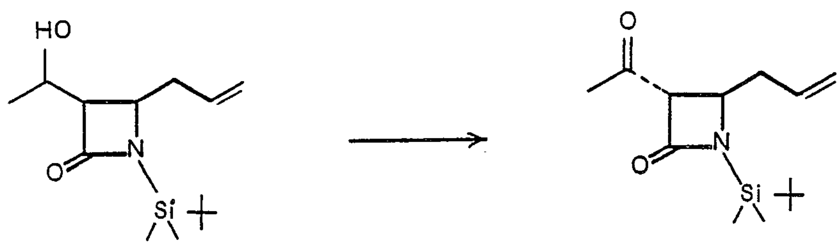

*A*. Trifluoroacetic anhydride (7.5 mmol) is added dropwise by syringe to a solution of dimethylsulfoxide (10 mmol) in anhydrous methylene chloride (15 ml) at −78°C. The resulting mixture is stirred at −78°C for 20 min. during which time a white precipitate forms. A solution of 1 - (t - butyldimethylsilyl) - 3 - (1 - hydroxyethyl) - 4 - (prop - 2 - ene) - azetidin - 2 - one (5.0 mmol) in methylene chloride (15 ml) is added by syringe and the resulting solution is stirred at −78°C for 30 min. Triethylamine (14 mmol) is added by syringe and the cooling bath is removed. After an additional 1 hr., the reaction mixture is diluted with methylene chloride (100 ml), washed with water (50 ml) and brine and dried over magnesium sulfate. Removal of solvents *in vacuo* yields an oil which is chromatographed on silica gel (2:1, petroleum ether:ether) to yield (3*S**,4*R**) - 1 - (t - butyldimethylsilyl - 3 - (1 - oxoethyl) - 4 - (prop - 2 - ene) - azetidin - 2 - one. I.R. ($CHCl_3$) 2925, 2855, 1734, 1705 $cm^{-1}$; n.m.r. ($CDCl_3$) δ 4.8—6.1 (3H, m, olefinic), δ 3.8—4.2 (2H, overlapping multiplets, H5, H6), δ 2.0—2.9 (2H, m, allylic),

$$\delta\ 2.3\ (3H,\ S,\ CH_3\text{—}\overset{O}{\overset{\|}{C}}),$$

δ 0.96 (9H, S, ± Si—), δ 0.25 (6H, 2S, $(CH_3)_2Si$). Mass spectrum m/e 267 (m+) 252, 226, 210.

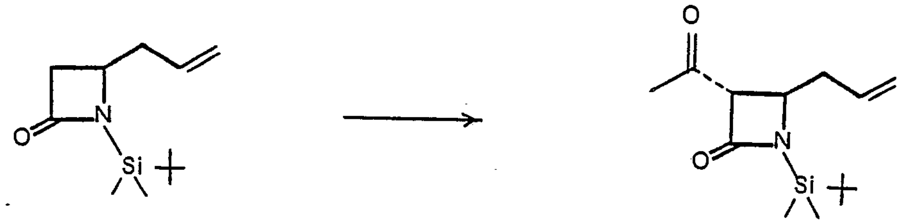

*B.* n-Butyllithium in hexane (4.10 mmol) is added by syringe to a solution of diisopropylamine (4.10 mmol) in anhydrous tetrahydrofuran (16 ml) at −78°C. The resulting solution is stirred at −78°C for 15 min. prior to the addition of a solution of 1 - (t - butyldimethylsilyl - 4 - (prop - 2 - ene) - azetidin - 2 - one (2.0 mmol) in anhydrous tetrahydrofuran (2 ml). After an additional 15 min. at −78°C, the reaction mixture is added *via* a Teflon tube to a mixture of N-acetylimidazole (4.1 mmol) in anhydrous tetrahydrofuran (16 ml) at −78°C. The resulting yellow reaction mixture is stirred at −78°C for 15 min., then quenched by addition of saturated aqueous ammonium chloride solution (10 ml). The reaction mixture is diluted with ether (100 ml) and washed with 2.5N hydrochloric acid solution (25 ml) water (25 ml) and brine. The organic phase is dried over magnesium sulfate and concentrated *in vacuo* to yield an oil. This material is chromatographed on silica gel (2:1 petroleum ether:ether) to yield (3*S**,4*R**) - 1 - (t - butyldimethylsilyl) - 3 - (1 - oxoethyl) - 4 - (prop - 2 - ene) - azetidin - 2 - one.

Example 4

(3*S**, 4*R**)-1-(t-Butyldimethylsilyl)-3-[(*R**)-1-hydroxyethyl]-4-(prop-2-ene)-azetidin-2-one

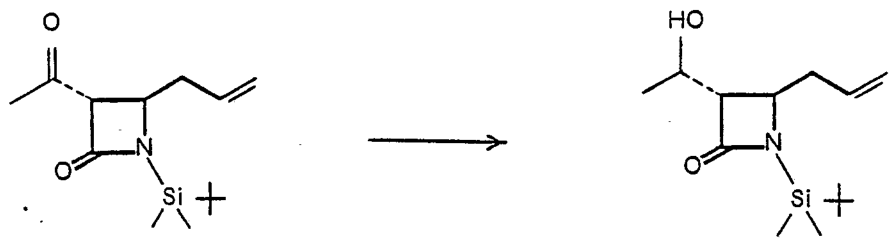

K-Selectride (potassium tri-(sec)-butylborohydride) in tetrahydrofuran (4.8 mmol) is added by syringe to a mixture of potassium iodide (2.0 mmol) and (3*S**, 4*R**) - 1 - (t - butyldimethylsilyl) - 3 - (1 - oxoethyl) - 4 - (prop - 2 - ene) - azetidin - 2 - one (2.0 mmol) in anhydrous ether (20 ml) at room temperature. The resulting mixture is stirred at room temperature for 2.5 hours, then quenched by addition of glacial acetic acid (9.6 mmol). The resulting mixture is diluted with ethylacetate (100 ml) and filtered through celite. Removal of solvents *in vacuo* gives an oil which is chromatographed on silica gel (1:1 ether:petroleum ether) to yield 1.90 g (95%) of (3*S**, 4*R**) - 1 - (t - Butyldimethylsilyl) - 3 - (1 - hydroxyethyl) - 4 - (prop - 2 - ene) - azetidin - 2 - one as a white solid. NMR examination of this material indicates the R*/S* ratio at the hydroxy center to be >5/1. The *R** isomer is isolated by crystallization from petroleum ether.

Example 5

(3*S**, 4*R**)-1-(t-Butyldimethylsilyl)-3-[(*R**)-1-hydroxyethyl]-4-(carboxymethyl)-azetidin-2-one

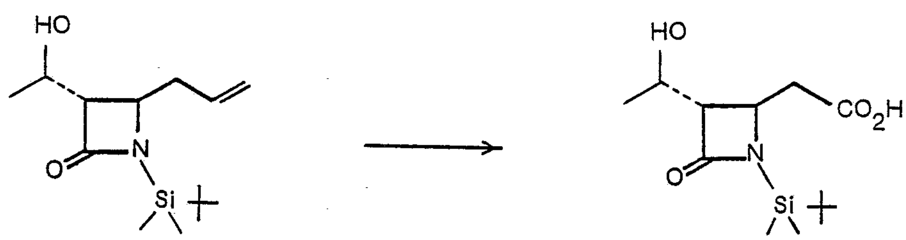

A solution of (3*S**, 4*R**) - 1 - (t - Butyldimethylsilyl) - 3 - [(*R**) - 1 - hydroxyethyl] - 4 - (prop - 2 - ene) - azetidin - 2 - one (3.0 mmol) in dry methylene chloride (30 ml) is cooled to −78°C (dry ice-acetone) and a stream of ozone is bubbled through until the reaction mixture becomes blue. The ozone flow is then stopped and the reaction is purged by bubbling through nitrogen until the blue color disappears. Solid m-chloroperbenzoic acid (3.0 mmol) is added and the cold bath is removed. When the reaction mixture

reaches room temperature, the flask is fitted with a reflux condenser and the mixture is heated at reflux for three days. Removal of solvents *in vacuo* gives a white solid which is chromatographed on silica gel (2% glacial acetic acid in methylene chloride) to yield 663 mg (77%) of (3*S**, 4*R**) - 1 - (t - Butyldimethylsilyl) - 3 - [(*R**) - 1 - hydroxyethyl] - 4 - (carboxymethyl) - azetidin - 2 - one. n.m.r. ($CDCl_3$) + $D_2O$) $\delta$ 3.6—4.3 (2H, m), $\delta$ 2.98 (1H, dd, J=7, 2.1), $\delta$ 2.7 (2H, d, of ABq, —$CH_2CO_2H$), $\delta$ 1.29 (3H, d, J=6), $\delta$ 0.95 (9H, S, $\delta$0.25 (6H, S).

Example 6

(3*S**, 4*R**)-1-(t-Butyldimethylsilyl)-3-[(*R**)-1-hydroxyethyl]-4-(3-p-nitrobenzyloxycarbonyl-2-oxopropyl)-azetidin-2-one

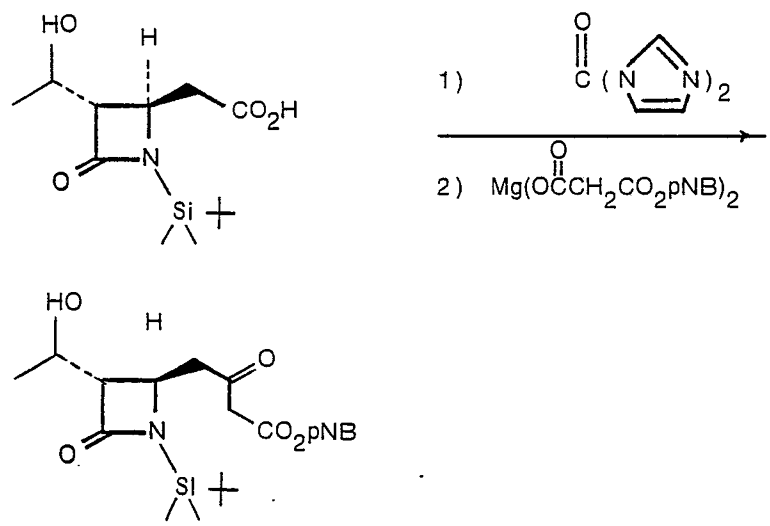


1,1'-Carbonyldimidazole (1.10 mmol) is added in one portion to a solution of (3*S**, 4*R**) - 1 - (t - butyldimethylsilyl) - 3 - [(*R**) - 1 - hydroxyethyl] - 4 - carboxymethyl - azetidin - 2 - one (1.0 mmol) in anhydrous tetrahydrofuran (5 ml) at room temperature. The resulting solution is stirred at room temperature for 6 hours. In a second flask, magnesium ethoxide (5 mmol) is added in one portion to a solution of the mono-p-nitrobenzyl ester of malonic acid (10 mmol) in anhydrous tetrahydrofuran (25 ml). The resulting mixture is stirred at room temperature for 1 hr, then the tetrahydrofuran is removed at the pump and the gummy residue is triturated with ether to yield the magnesium salt as an off-white solid. (1.1 mmol) of this magnesium salt is then added to the first reaction flask and the resulting mixture is stirred at room temperature for 18 hrs. The reaction mixture is then poured into 50 ml of ether, washed with 0.5N hydrochloric acid solution (20 ml), water (20 ml), saturated aqueous sodium bicarbonate solution (20 ml), brine and dried over magnesium sulfate. Removal of solvents *in vacuo* gives an oil which is chromatographed on silica gel (ether) to yield (3*S**, 4*R**) - 1 - (t - butyldimethylsilyl) - 3 - [(*R**) - 1 - hydroxyethyl] - 4 - (3 - p - nitrobenzyloxycarbonyl - 2 - oxopropyl) - azetidin - 2 - one. n.m.r. ($CDCl_3$—$H_2O$) $\delta$ 8.24, 8.10, 7.52, 7.38 (2H, AB, aromatic), $\delta$ 5.26 (2H, S, —$CH_2$—Ar, $\delta$ 3.5—4.2 (2H, m, H—5, H—8),

$$\delta\ 2.6—3.3\ (3H, m, H—6, —CH_2—\overset{O}{\overset{\|}{C}}—),$$

$\delta$ 1.3 (3H, d, J=6.6, $CH_3$—), $\delta$ 0.98 (9H, S, ± Si—), $\delta$ 0.25 (6H, S, $(CH_3)_2Si$<).

Example 7

(3*S**,4*R**)-3-[(*R**)-1-hydroxyethyl]-4-(3-p-nitrobenzyloxycarbonyl-2-oxopropyl)-azetidin-2-one

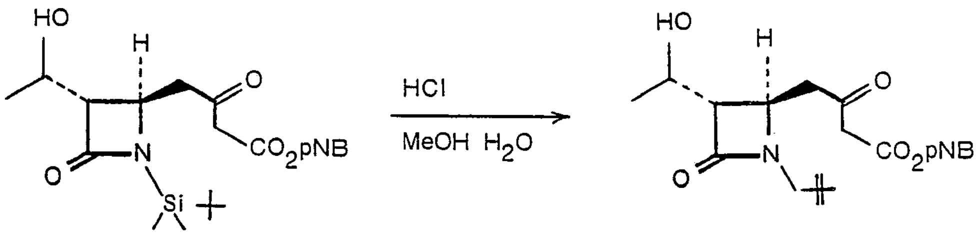

A solution of (3$S$*, 4$R$*) - 1 - (t - Butyldimethylsilyl) - 3 - [($R$*) - 1 - hydroxyethyl] - 4 - (3 - p - nitrobenzyloxycarbonyl - 2 - oxopropyl) - azetidin - 2 - one (1.0 mmol) in 20 ml of 9:1 (v/v) methanol-water is cooled to 0°C. Concentrated hydrochloric acid (0.34 ml) is added and the resulting solution is stirred at 0°C for 15 min., then allowed to warm to room temperature. After 2.5 hrs., at room temperature the reaction mixture is diluted with ethyl acetate (25 ml), washed with water (10 ml) and brine, dried over magnesium sulfate and concentrated *in vacuo* to yield (3$S$*, 4$R$*) - 3 - [($R$*) - 1 - hydroxyethyl] - 4 - (3 - p - nitrobenzyloxycarbonyl - 2 - oxopropyl) - azetidin - 2 - one.

Example 8

Prepration of (3$S$*,4$R$*)-3-[R*)-1-hydroxyethyl)-4-[3-(4-nitrobenzyl)oxycarbonyl-2-oxo-3-diazopropyl]-azetidin-2-one

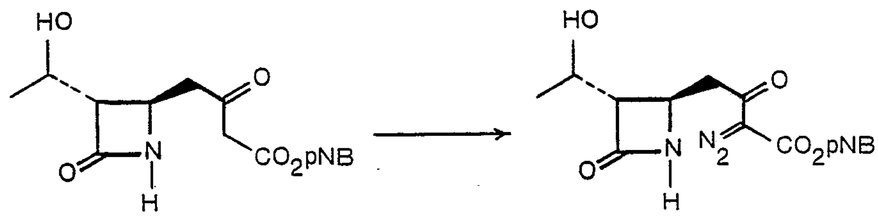

Triethylamine (263 mg, 2.6 mmol) is added by syringe to a mixture of (3$S$*,4$R$*) - 3 - [($R$*) - 1 - hydroxyethyl] - 4 - [3 - (4 - nitrobenzyl)oxycarbonyl - 2 - oxopropyl] - azetidin - 2 - one (253 mg, 0.72 mmol) and p-carboxybenzene sulfonylazide (196 mg, 0.84 mmol) in dry acetonitrile (6 ml) at 0°C. When addition is complete the cooling bath is removed and the reaction mixture is stirred at room temperature for 1 hour. The mixture is then diluted with ethyl acetate (50 ml) and filtered. The filtrate is concentrated *in vacuo* and the residue is chromatographed on a short silica gel column (ethyl acetate) to yield 222 mg, (81% overall from (3$S$*, 4$R$*) - 1 - (t - Butyldimethylsilyl) - 3 - [($R$*) - 1 - (t - butyldimethylsilyloxy)ethyl] - 4 - [3 - (4 - nitrobenzyl)oxycarbonyl - 2 - oxopropyl]azetidin - 2 - one) of (3$S$*, 4$R$*) - 3 - (R*) - 1 - hydroxyethyl) - 4 - [3 - (4 - nitrobenzyl)oxycarbonyl - 2 - oxo - 3 - diazopropyl]azetidin - 2 - one as a white solid. IR ($CHCl_3$, $CM^{-1}$) 3410, 2132, 1756, 1718, 1650, 1350, 1280, 1120; n.m.r. ($CDCl_3$) δ 7.9 (2d-aromatic, 4), δ 5.4 (s, 2), δ 6.2 (brs, 1), δ 4.1 (m, 2), δ 2.6—3.6 (m, 4), δ 1.32 (d, 3, J=6.2).

Example 9

Preparation of (5$R$*,6$S$*)p-Nitrobenzyl 6-[($R$*)-1-hydroxyethyl]-1-azabicyclo[3.2.0]heptan-3,7-dione-2-carboxylate

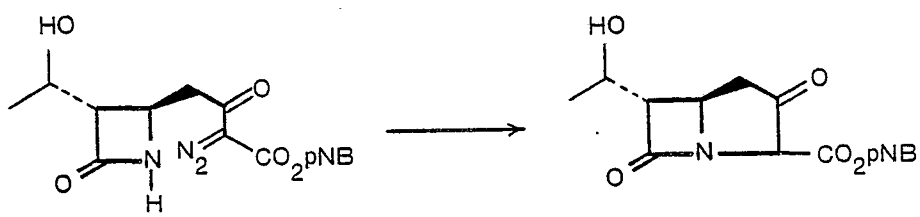

A suspension of (3$S$*, 4$R$*) - 3 - [(R*) - 1 - hydroxyethyl] - 4 - [3 - (4 - nitrobenzyl)oxycarbonyl - 2 - oxo - 3 - diazopropyl]azetidin - 2 - one (56.4 mg, 0.15 mmol) and rhodium (II) acetate (0.1 mg) in dry benzene (3 ml) is deoxygenated by bubbling through nitrogen for 10 minutes. The mixture is then heated to 78°C for 1 hour. During heating the solid starting material gradually goes into solution. The mixture is then cooled, filtered to remove the catalyst, and the filtrate is concentrated *in vacuo* to yield (5$R$*, 6$S$*) - p - nitrobenzyl 6 - [(R*) - 1 - hydroxyethyl] - 1 - azabicyclo[3.2.0]heptan - 3,7 - dione - 2 - carboxylate.

Physical Properties:

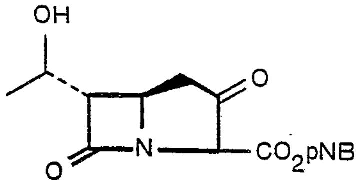

pNB = p-nitrobenzyl

n.m.r.: (300MHz, $CDCl_3$) δ 8.26, 7.54 (aromatic, 4), 5.29 (AB, 2), 4.77 (s, 1), 4.32 (dg, 1, J=6.6, 7), 4.16 (ddd, 1, J=7, 7.5, 2.2), 3.21 (dd, 1, J=7, 2.2), 2.94 (dd, 1, J=19.5, 7), 2.50 (dd, 1, J=19.5, 7.5), 2.2 (brs, 1), 1.37 (d, 3, J=6.6). I.R.: ($CHCl_3$, $CM^{-1}$) 1770, 1758, 1610, 1522, 1353.

Example 10

Preparation of *p*-Nitrobenzyloxycarbonylaminoethanethiol

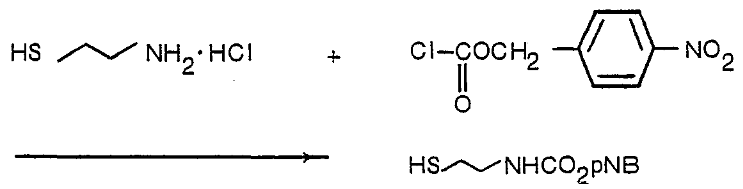

To 600 ml diethyl ether ($Et_2O$) — 75 ml $H_2O$ in an ice bath with stirring is added 3.2 g cysteamine hydrochloride (mw = 114; 28.1 mmole). A solution of 7.14 g $NaHCO_3$ (mw = 84; 85 mmol) in 75 ml $H_2O$ is added. The ice bath is removed, and at room temperature a solution of 6.75 g *p*-nitrobenzylchloroformate (mw = 216; 31.3 mmol) in 270 ml $Et_2O$ is added dropwise over a period of one hour. After 10 additional minutes, the layers are separated. The ether layer is extracted with 150 ml 0.25 N HCl, and then with 200 ml brine. Each aqueous layer is then backwashed successively with 100 ml $Et_2O$. The combined $Et_2O$ layers are dried over anhydrous $MgSO_4$, filtered, and concentrated under a $N_2$ stream. The crystalline residue is slurried in a small amount of ether, filtered, and the pale yellow crystals are dried under high vacuum to give 4.7 g. *p*-nitrobenzyloxycarbonylaminoethanethiol (65% yield). NMR ($CDCl_3$): 8.18 (*d*, J=8Hz, aromatic protons ortho to nitro), 7.47 (*d*, J=8Hz, aromatic protons meta to nitro), 5.27 (—N$\underline{H}$—), 5.20 (*s*, —C$\underline{H}_2$—NH—), 2.67 (*m*, —C$\underline{H}_2$—SH), 1.35 (*t*, J=8.5Hz, —S$\underline{H}$) in ppm downfield from TMS. IR ($CHCl_3$ solution): carbonyl- 1725 $cm^{-1}$. M.S.: molecular ion-256, (M—47) at 209, (M—136) at 120, $^{+}CH_2\phi pNO_2$ at 136

Example 11

Preparation of (5*R**,6*S**)-p-Nitrobenzyl 3-[2-(p-nitrobenzyloxycarbonyl)aminoethylthio]-6-[(R*)-1-hydroxyethyl]-1-azabicyclo[3.2.0]hept-2-en-7-one-2-carboxylate

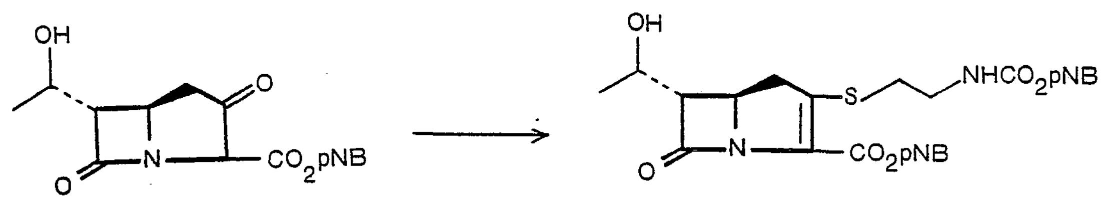

(5*R**,5*S**) - p - Nitrobenzyl 6 - [(R*) - 1 - hydroxyethyl] - 1 - azabicyclo[3.2.0]heptan - 3,7 - dione - 2 - carboxylate (51 mg, 0.147 mmol) is dissolved in acetonitrile (3 ml) and the resulting solution is cooled to 0°C. Diisopropylethylamine (22 mg, 0.17 mmol) is added by syringe and the resulting solution is stirred at 0°C for 1 minute prior to the addition of a solution of freshly recrystallized p-toluene sulfonic anhydride (51 mg, 0.156 mmol) in dry acetonitrile (1 ml). The resulting solution is stirred at 0°C for 1 hour to provide (5*R**,6*S**) - p - nitrobenzyl 3 - (p - toluenesulfonyloxy) - 6 - [(R*) - 1 - hydroxyethyl] - 1 - azabicyclo-[3.2.0]hept - 2 - en - 7 - one - 2 - carboxylate, then cooled to −25°C. Diisopropylethylamine (80.5 mg, 0.624 mmol) is added by syringe followed shortly thereafter by a solution of N - p - nitrobenzyloxycarbonylcysteamine (40 mg, 0.156 mmol) in 1 ml of dry acetonitrile. The reaction mixture is then stored in a refrigerator for 70 hrs. The mixture is diluted with 25 ml of ethylacetate washed with brine and dried over magnesium sulfate. Solvents are removed *in vacuo* to yield a yellw oil which is chromatographed on a silica gel plate (ethyl acetate, $R_f$ = 0.4) to yield (5*R**,6*S**) - p - nitrobenzyl - 3 - [2 - (p - nitrobenzyloxycarbonyl)aminoethylthio] - 6 - [(R*) - 1 - hydroxyethyl] - 1 - azabicyclo[3.2.0]hept - 2 - en - 7 - dione - 2 - carboxylate as a yellow solid. IR (Nujol mull) 1733 and 1690 $cm^{-1}$; n.m.r. ($CDCl_3$ δ 7.54—8.26 (overlapping ABq, 4), δ 5.40 (ABq, 2), δ 5.22 (s, 2), δ 4.27 (m, 2), δ 3.47 (m), 3.23 (dd, 1), δ 3.14 (dd, 1), δ 3.40 (dd, 1), δ 3.04 (m, 2), δ 1.37 (d, 3).

Example 12

Preparation of Thienamycin

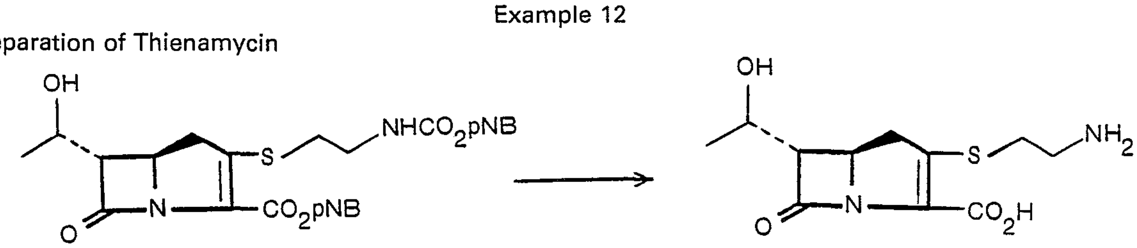

A mixture of N-p-nitrobenzyloxycarbonyl thienamycin p-nitrobenzyl ester (10 mg, 0.017 mmol) and 10% Pd/C—Bolhofer type in tetrahydrofuran (2 ml), 0.1M dipotassium hydrogen phosphate solution (1.4 ml) and 2-propanol (0.2 ml) is hydrogenated at 40 psi on the Parr shaker for 30 minutes. The mixture is then filtered and the catalyst is washed with water (3 × 3 ml). The combined filtrate and washings are extracted with ethyl acetate-ethyl ether then concentrated to ~3 ml and lyophilized.

Example 13

Preparation of 6-Methylthienamycin

* * *

1-(*t*-Butyldimethylsilyl)-3-(1-hydroxyethyl)-3α,β-methyl-4-(prop-2-en)-azetidin-2-one

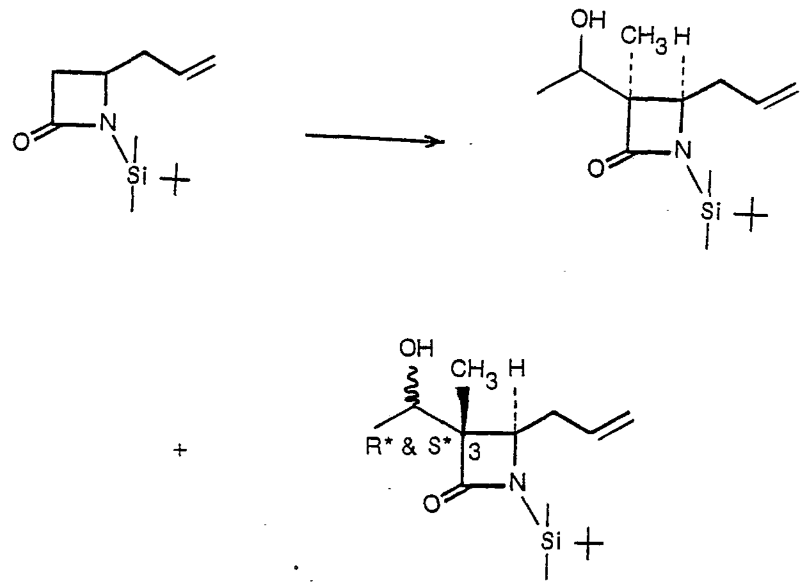

To 342 μl diisopropylamine (2.44 mmol) in 5 ml anhydrous tetrahydrofuran (THF) at −78°C with stirring under $N_2$ is added 1 ml of 2.45M n-butyllithium (2.45 mmol). After stirring for 10 min, a solution of 500 mg of 1 - (*t* - butyldimethylsilyl) - 4 - (prop - 2 - en) - azetidin - 2 - one in 2 ml anhydrous THF is added. After stirring for 10 min, 153 μl methyliodide (2.46 mmol) is added. The cooling bath is removed, and the reaction mixture is stirred for 25 min. Upon re-cooling to −78°C, 1 ml of 2.45M n-butyllithium (2.45 mmol) is added. After 5 min, 250 μl distilled acetaldehyde (4.49 mmol) is added, and the cooling bath is removed. After 30 min, the reaction mixture is added to 10 ml 1M $KH_2PO_4$-brine-$Et_2O$. After separation of the layers, the organic layer is washed with 10 ml 1M $KH_2PO_4$-15 ml brine and then with brine. After drying over magnesium sulfate, filtration, and concentration *in vacuo*, 626 mg of crude reaction product is obtained. Chromatography on silica gel (0—20% ether:petroleum ether) provides 125 mg of the 3α-methyl compound and 234 mg of a slightly more polar mixture of 3β-methyl compounds (*R** and *S**).

NMR of 3α methyl component (300MHz, $CDCl_3$) δ 0.24 and 0.26 (2S, $Si(CH_3)_2$), 0.97 (S, Si—$C(CH_3)_3$), 1.22 (d, J=6Hz, $CH_3CHOH$—), 1.30 (S, $CH_3$), 2.34 (d, OH), 2.42—2.81 (m, $CH_2CH{=}CH_2$), 3.44 (dd, J=4 and 10Hg, $H_4$), 4.09—4.19 (m, $CH_3CHOH$—), 5.09—6.02 (m, —$CH{=}CH_2$).

NMR of 3β-methyl components (300MHz, $CDCl_3$) δ 0.23—0.25 (s's, $Si(CH_3)_2$ 'S), 0.95 and 0.96 (2S, Si—$C(CH_3)_3$ 'S), 1.14—1.22 (series of peaks for $CH_3$ 's and $CH_3CHOH$ 'S), 2.29—2.62 (m, $CH_2CH{=}CH_2$), 3.40 (dd, J=3.5 and 11Hz, $H_4$ of *S**), 3.70 (dd, J=3.5 and 11Hz, $H_4$ of *R**), 3.86—4.00 (m, $CH_3CHOH$—), 5.08—5.96 (m, CH—$CH_2$).

1-(*t*-Butyldimethylsilyl)-3-(1-oxoethyl)-3β-methyl-4-(prop-2-en)-azetidin-2-one

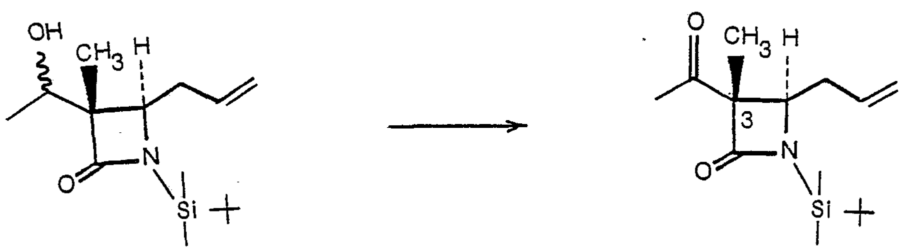

Trifluoroacetic anhydride (180 µl, 1.28 mmol) is added dropwise by syringe to a solution of dimethylsulfoxide (120 µl, 1.69 mmol) in anhydrous methylene chloride (2.5 ml) at −78°C under $N_2$. The resulting mixture is stirred at −78°C for 15 min. A solution of 1 - (t - butyldimethylsilyl) - 3 - (1 - hydroxyethyl) - 3β - methyl - 4 - (prop - 2 - en) - azetidin - 2 - one (234 mg, 0.83 mmol) in methylene chloride (2 ml) is added, and the resulting solution is stirred at −78°C for 1 hr. Triethylamine (382 µl, 2.76 mmol) is added by syringe, and the cooling bath is removed. After an additional 1 hr, the reaction mixture is diluted with methylene chloride, washed with water and brine, dried over magnesium sulfate, filtered. Removal of solvents *in vacuo* yields partially crystalline oil. Chromatography on silica gel (0—10% ether:petroleum ether) provides 191 mg of the title compound.

IR ($CHCl_3$) µ 5.76, 5.87

MS m/e NoM$^+$, 266, 240, 224

NMR (60MHz, $CDCl_3$) 0.25 (2S, $Si(CH_3)_2$), 0.97 (s, Si—$C(CH_3)_3$), 1.45 (s, $CH_3$), 2.28 (s, $CH_3$—C=O), 2.33—2.62 (m, $C\underline{H}_2CH=CH_2$), 4.15 (dd, J=5 and 10Hz, $H_4$), 4.88—6.10 (m, $C\underline{H}=C\underline{H}_2$).

1-(*t*-Butyldimethylsilyl)-3-(1-hydroxyethyl)-3β-methyl-4-(prop-2-en)-azetidin-2-one

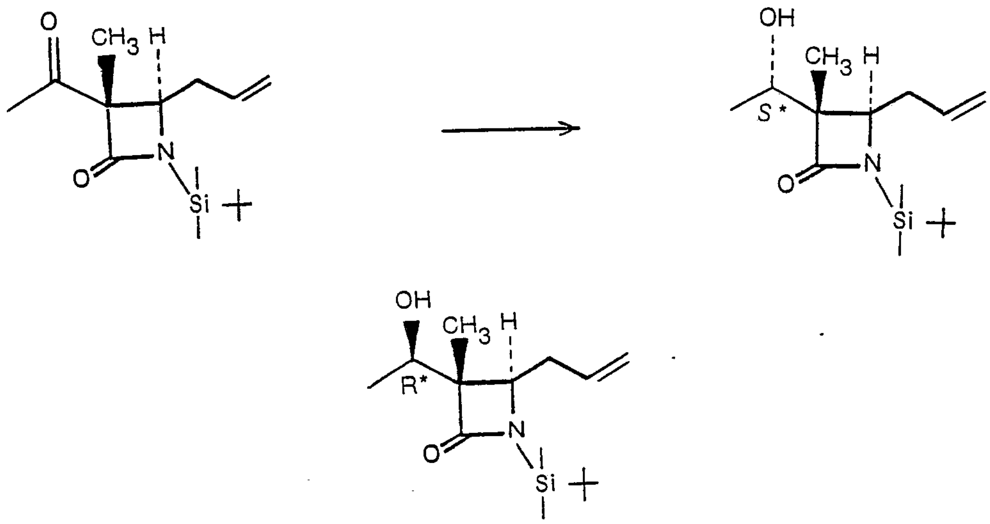

To a stirred solution of 4.26 g of 1 - (*t* - butyldimethylsilyl) - 3 - (1 - oxoethyl) - 3β - methyl - 4 - (prop - 2 - en) - azetidin - 2 - one (15.2 mmol) in 90 ml *i*-propanol is added 807 mg sodium borohydride (21.2 mmol). After stirring vigorously under $N_2$ for 45 min, the reaction mixture is poured (carefully) into ~100 ml 1M $KH_2PO_4$— 400 ml $H_2O$—500 ml $Et_2O$. After phase separation, the aqueous layer is washed 2 × $Et_2O$. The combined organic layers are then washed 2 × brine, dried over magnesium sulfate, filtered, and concentrated *in vacuo* to 4.1 g of an oil. Chromatography on 300 g silica gel (0—10% acetone:hexane) provided 3.84 g of material containing mainly the *S** diastereomer and 1.58 g of the R* diastereomer. Data for the *R** diastereomer:

IR ($CHCl_3$) µ 5.80

MS m/e 284 (M$^+$ + 1), 268, 226

NMR (300 MHz, $CDCl_3$) δ 0.24 (2S, $Si(CH_3)_2$, 0.96 (s, $Si(CH_3)_3$), 1.17 (d, partially hidden by sat 1.18, $C\underline{H}_3CHOH$—), 1.18 (s, $CH_3$) 1, 1,70 (d, OH), 2.32—2.60 (m, $C\underline{H}_2CH=CH_2$), 3.71 (dd, J=4 and 11Hz, $H_4$), 3.89—3.98 (m, $CH_2C\underline{H}OH$), 5.12—5.90 (m, $C\underline{H}=C\underline{H}_2$).

1-(*t*-Butyldimethylsilyl)-3-(1-*t*-butyldimethylsilyloxyethyl)-3β-methyl-4-(prop-2-en)-azetidin-2-one

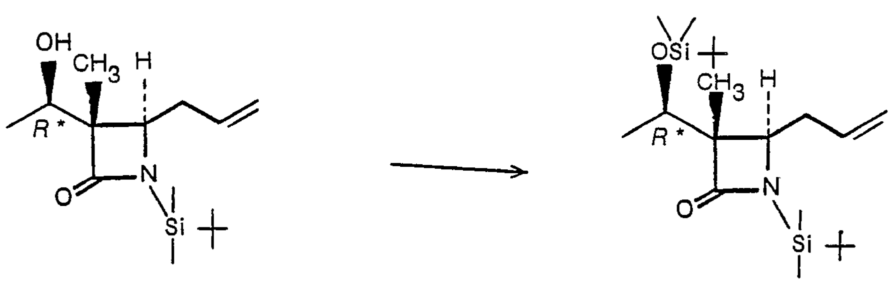

With stirring under $N_2$, 582 mg of 1 - (*t* - butyldimethylsilyl) - 3 - (1 - hydroxyethyl) - 3β - methyl - 4 - (prop - 2 - en) - azetidin - 2 - one (2.06 mmol) in 6 ml anhydrous N,N-dimethylformamide is treated with 320 µl triethylamine (2.31 mmol) followed by 356 mg *t*-butyldimethylsilylchloride (2.37 mmol). After

stirring over night, the reaction mixture is poured into 1.3 ml, 1M$KH_2PO_4$— 50 ml brine-50 ml methylene chloride. After phase separation, the aqueous layer is again extracted with methylene chloride. The combined organic layers are washed 2 × brine, dried over magnesium sulfate, filtered and concentrated to 699 mg crude material. Chromatography on silica gel (0—50% ethylacetate:hexane) provides 473 mg of the title compound and 211 mg recovered starting material.

IR ($CHCl_3$) μ 5.78

MS m/e 397 ($M^+$), 382, 340

NMR (300MHz, $CDCl_3$) δ 0.05, 0.08, 0.21 and 0.26 (4S, $Si(CH_3)_2$'s), 0.90 and 0.96 (2S, Si—$C(CH_3)_3$'s), 1.11 (d, J=6Hz, $CH_3CHOSi$—), 1.14 (S, $CH_3$), 2.30—2.56 (m, $CH_2CH=CH_2$), 3.66 (dd, J=4 and 10Hz, $H_4$), 3.91 (q, J=6Hz, CHCHOSi—), 5.08—5.94 (m, —CH=$CH_2$).

1-(t-Butyldimethylsilyl)-3-(1-*t*-butyldimethylsilyloxyethyl)-3β-methyl-4-(2-oxoethyl)-azetidin-2-one

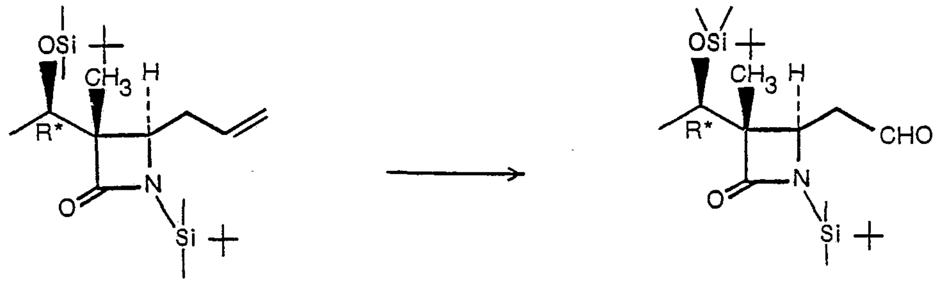

A solution of 1.05 g (1 - (*t* - butyldimethylsilyl) - 3 - (1 - *t* - butyldimethylsilyloxyethyl) - 3β - methyl - 4 - (prop - 2 - en) - azetidin - 2 - one (2.6 mmol) in 30 ml anhydrous methylene chloride is cooled to −78°C, and a stream of dried ozone in oxygen is bubbled through until the blue color disappears. The cooling bath is removed, and the reaction mixture is concentrated under a stream of $N_2$ and then *in vacuo*. The crude ozonide is dissolved in 5 ml methylene chloride and is treated with 580 μl dimethylsulfide (7.9 mmol) and placed under $N_2$. After 6 hrs, 580 μl additional dimethylsulfide is added, and the reaction is allowed to stir overnight under $N_2$. The reaction is then concentrated under high vacuum followed by chromatography on silica gel (methylene chloride). Forward fractions, containing unreacted ozonide as well as the desired aldehyde, are re-treated with dimethylsulfide to increase the yield. Later fractions contain 531 mg of the desired aldehyde.

IR ($CHCl_3$) μ 5.75, 5.80 (sh)

M.S. m/e 400 ($M^+$ + 1), 384, 342

NMR (300 MHz, $CDCl_3$) δ 0.06, 0.09, 0.19 and 0.24 (4S, $Si(CH_3)_2$'s), 0.88 and 0.95 (2S, $Si(CH_3)_3$'s), 1.09 (s, $CH_3$), 1.15 (d, J=6Hz, $CH_3CHOSi$—), 2.75—2.80 (m, $CH_2CHO$), 3.98 (q, J=6Hz, $CH_3CHOSi$—), 4.16 (dd, J=5.5 and 7.5 Hz, $H_4$), 9.84 (t, CHO).

1-(t-Butyldimethylsilyl)-3-(1-*t*-butyldimethylsilyloxyethyl)-3β-methyl-4-(carboxymethyl)-azetidin-2-one

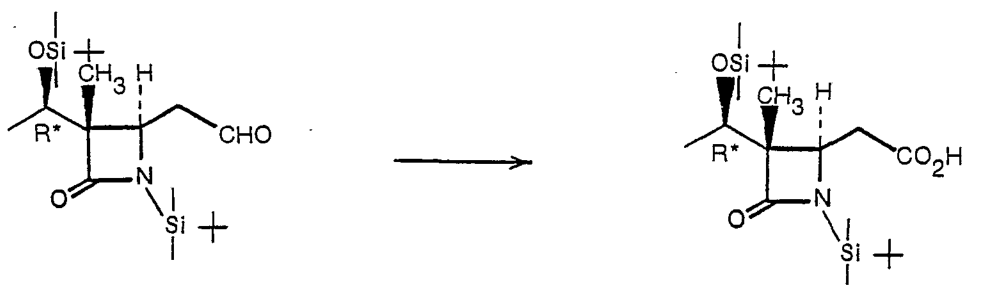

A solution of 620 mg 1 - (*t* - Butyldimethylsilyl) - 3 - (*t* - butyldimethylsilyloxyethyl) - 3β - methyl - 4 - (2 - oxoethyl) - azetidin - 2 - one (1.55 mmol) in 22 ml distilled acetone is cooled in an ice bath and treated with 447 μl of Jones reagent (2.6M in $CuO_3$, 1.16 mmol). After stirring for 15 min., 409 μl absolute ethanol is added, and stirring is continued for 5 min. The cooling bath is removed, and the reaction mixture is quickly concentrated under a $N_2$ stream to a small volume. Ethylacetate and water are added to the concentrate. After phase separation, the organic layer is washed 3 × brine, dried over magnesium sulfate, filtered, and concentrated to 629 mg crude product. Chromatography on silica gel (0—1% acetic acid:methylene chloride) provides 498 mg of the desired product.

IR ($CHCl_3$) μ 5.76 br

M.S. m/e (silylated)- NoM$^+$ at 487, 472, 430 (487-*t*-butyl)

NMR (300MHz, $CDCl_3$) δ0.04, 0.07, 0.20 and 0.24 (4S, $Si(CH_3)_2$'s, 0.86 and 0.93 (2s, Si—$C(CH_3)_3$'s), 1.09 (d, J=6Hz, $\underline{CH_3}$CHOSi), 1.11 (s, $CH_3$), 2.63 and 2.72 (2dd, J=9 and 16Hz and J=5 and 16Hz respectively, $\underline{CH_2}CO_2H$), 3.96 (q, J=6Hz, $CH_3\underline{CH}$OSi—), 4.11 (dd, J=5 and 9Hz, $H_4$).

1-(t-Butyldimethylsilyl)-3-(1-*t*-butyldimethylsilyloxyethyl)-3β-methyl-4-(3-p-nitrobenzyloxycarbonyl-2-oxopropyl)azetidin-2-one

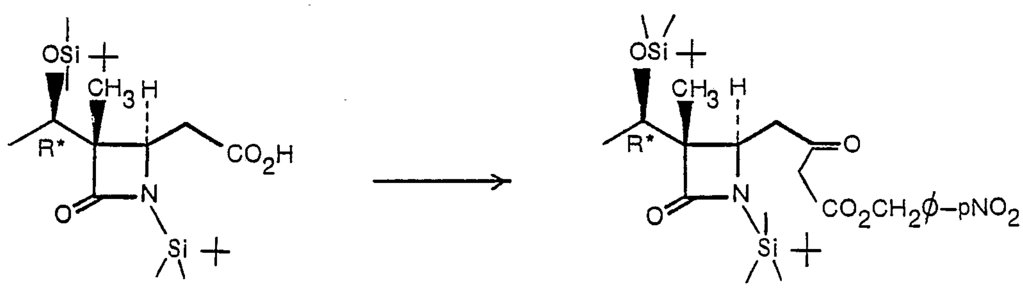

1,1'-Carbonyldiimidazole (1.09 mg, 0.67 mmol) is added in one portion to a solution of 251 mg 1 - (*t* - butyldimethylsilyl) - 3 - (1 - *t* - butyldimethylsilyloxyethyl) - 3β - methyl - 4 - (carboxymethyl) - azetidin - 2 - one (0.60 mmol) in anhydrous tetrahydrofuran (3 ml) at room temperature under $N_2$. The resulting solution is stirred at room temperature for 3.5 hours. In a second flask, magnesium ethoxide (5 mmol) is added in one portion to a solution of the mono-p-nitrobenzyl ester of malonic acid (10 mmol) in anhydorus tetrahydrofuran (25 ml). The resulting mixture is stirred at room temperature for 1 hr, then the tetrahydrofuran is removed at the pump, and the gummy residue is triturated with ether to yield the magnesium salt as an off-white solid. The magnesium salt (339 mg, 0.678 mmol) is then added to the first reaction flask and the resulting mixture is stirred at room temperature overnight. The reaction mixture is then poured into 50 ml of ether, washed with 0.5N hydrochloric acid solution (12.3 ml), water (12.3 ml), saturated aqueous sodium bicarbonate solution (12.3 ml) , brine and dried over magnesium sulfate. Removal of solvents *in vacuo* gives 356 mg of an oil which is chromatographed on 6—1000 μ silica gel GF plates (25% acetone/hexane). The desired UV band is immediately scraped and extracted with 70% acetone/hexane. Concentration *in vacuo* gives 235 mg of the title compound.

IR ($CHCl_3$) μ 5.75

M.S. m/e NoM$^+$ at 592, 577, 535 (M$^+$-*t*-butyl)

NMR (300MHz, $CDCl_3$) δ 0.04, 0.08, 0.15 and 0.20 (4S, $Si(CH_3)_2$'s), 0.86 and 0.92 (2S, Si—$C(CH_3)_3$'s), 1.02 (s, $CH_3$), 1.12 (d, J=6Hz, $\underline{CH_3}$CHOSi—),

2.87 (brd, J=6Hz, 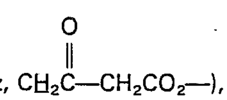),

3.58 (S, 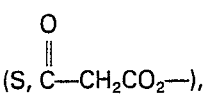),

3.95 (q, J=6Hz, $CH_3\underline{CH}$OSi—), 4.17 (brt, J=6Hz, $H_4$) 5.29 (s, $CO_2\underline{CH_2}$ØpNO$_2$), 7.56 and 8.27 (2d, aromatic protons).

3-(1-hydroxyethyl)-3β-methyl-4-(3-*p*-nitrobenzyloxycarbonyl-2-oxopropyl)azetidin-2-one

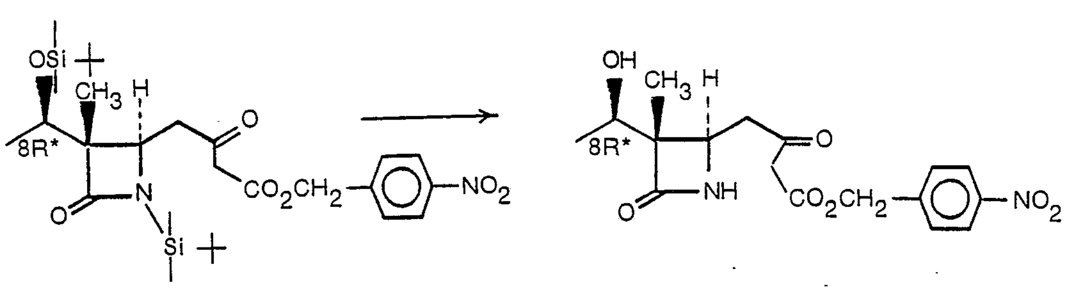

Concentrated hydrochloric acid (0.68 ml) is brought to a volume of 40 ml with 9:1 methanol:water, and 22 ml of the solution (4.5 mmol) is added to 319 mg of 1 - (*t* - butyldimethylsilyl) - 3 - (1 - *t* - butyldimethylsilyloxyethyl) - 3β - methyl - 4 - (3 - *p* - nitrobenzyloxycarbonyl - 2 - oxopropyl) - azetidin - 2 - one (0.54 mmol) with good stirring under $N_2$. After 8 hr, the reaction is added to 8 ml 1M $K_2HPO_4$ — 150 ml $H_2O$ — 150 ml ethylacetate. After phase separation, the organic layer is washed with brine containing 8 ml 1M $K_2HPO_4$ and then brine alone (2×). After drying over magnesium sulfate, filtration, and concentration *in vacuo*, 241 mg of an oil is obtained. Chromatography on 6—1000 μ silica gel GF plates (50% acetone/hexane) followed by extraction of the desired UV band with 70% acetone/hexane provides 129 mg of the title compound. A less polar UV band contains 57 mg of material still having the O-silyl group intact.

Data for the compound:
IR ($CHCl_3$) μ 5.70 (br), 5.81 (sh)
MS. m/e
NMR (300MHz, $CDCl_3$) δ 1.14 (s, $CH_3$), 1.23 (d, J=6Hz, $C\underline{H}_3CHOH$),

$$2.81—2.98\ (m,\ C\underline{H}_2\overset{\overset{O}{\|}}{C}CH_2CO_2—),$$

3.62 (s, $C\underline{H}_2CO_2$—$CH_2$ØpNO$_2$), 4.05 (center of m, $H_4$ and $CH_3CHOH$), 5.30 (s, $CO_2C\underline{H}_2$ØpNO$_2$), 6.01 (br, s, NH), 7.57 and 8.30 (2d, aromatics).

3-(1-Hydroxyethyl)-3β-methyl-4-(3-diazo-3-*p*-nitrobenzyloxycarbonyl-2-oxopropyl)-azetidin-2-one

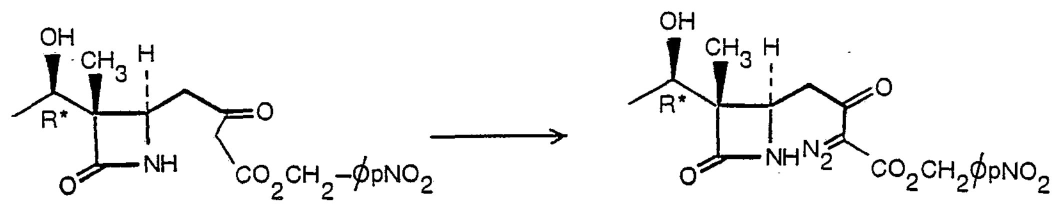

Triethylamine (55 μl, 0.397 mmol) is added by syringe to a mixture of 3 - (1 - hydroxyethyl) - 3β - methyl - 4 - [3 - (*p* - nitrobenzyl)oxycarbonyl - 2 - oxopropyl] - azetidin - 2 - one (40 mg, 0.11 mmol) and *p*-carboxybenzene sulfonylazide (30 mg, 0.13 mmol) in dry acetonitrile (1 ml) at 0°C. When addition is complete, the cooling bath is removed, and the reaction mixture is stirred at room temperature for 1.5 hour. The mixture is then diluted with ethyl acetate (10 ml) and filtered. The filtrate is concentrated *in vacuo*, and the residue is dissolved in methylene chloride, filtered, and concentrated *in vacuo* to 53 mg white foam. A fast filtration through a short silica gel column (ethylacetate) provides 41 mg of the title compound as a slightly off white solid. On a larger scale, the product may crystallize out of the reaction mixture along with by-products of the reaction. Extraction of the original, insoluble materials with methylene chloride, followed by chromatography of the contents of the filtrate, yields the product in these instances.

mp 162—170°C dec.
IR ($CHCl_3$) μ 4.70, 5.70, 5.81
NMR (300MHz, di-DMSO) δ 0.95 (s, $CH_3$), 1.03 (d, J=6Hz, $C\underline{H}_3CHOH$),

$$3.05\ (d,\ J{=}6Hz,\ C\underline{H}_2\overset{\overset{O}{\|}}{C}CH_2CO_2—),$$

3.68—3.76 (m, $CH_3CHOH$), 3.90 (t, J=6Hz, $H_4$), 4.85 (d, OH), 5.46 (s, $CH_2$—Ø—p—$NO_2$), 7.76 and 8.31 (2d, aromatic protons), 7.93 (s, NH).

p-Nitrobenzyl 6-(1-hydroxyethyl)-6β-methyl-3-[2-p-nitrobenzyloxycarbonyl)amino]ethylthio-1-azabicyclo-[3.2.0]hept-2-en-7-one-21-carboxylate

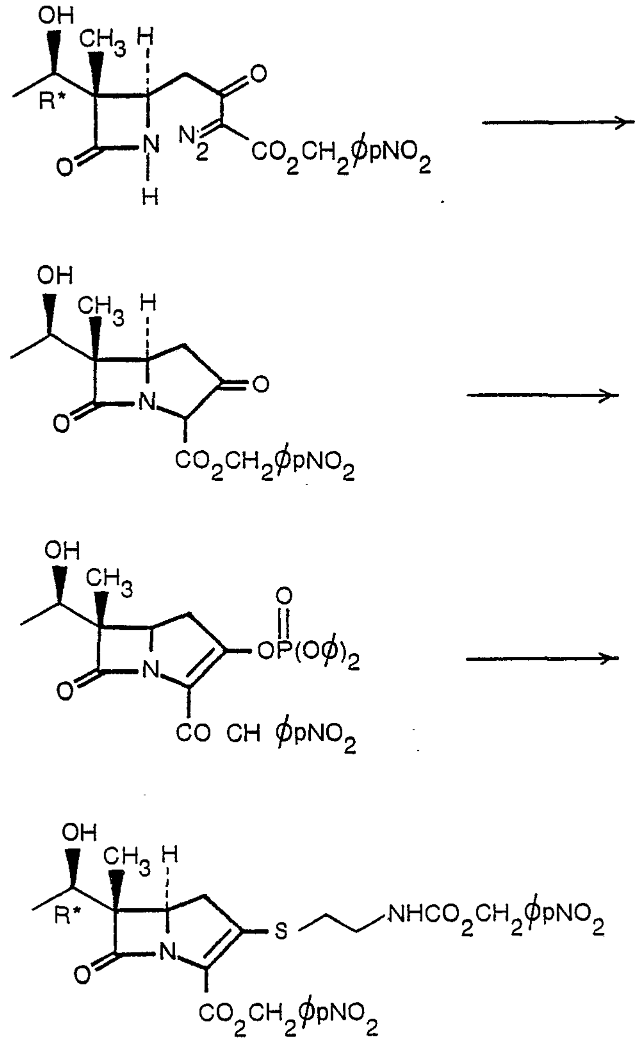

A suspension of 3 - (1 - hydroxyethyl) - 3β - methyl - 4 - [3 - diazo - 3 - p - nitrobenzyloxycarbonyl - 2 - oxopropyl] - azetidin - 2 - one (35 mg, 0.09 mmol) and rhodium (II) acetate (0.1 mg) in dry toluene (3.2 ml) is deoxygenated by evacuating and treating with nitrogen alternately (3×). The mixture is then heated to ca. 100°C for 1 hour with stirring under $N_2$. During heating the solid starting material gradually goes into solution. The mixture is then cooled, filtered to remove the catalyst , and the filtrate is concentrated *in vacuo* to yield the above bicycloketone as a white foam. The crude bicycloketone (32.5 mg, 0.09 mmol) is dissolved in anhydrous acetonitrile (1.8 ml), and the resulting solution is cooled to 0°C under $N_2$. Diisopropylethylamine (18.9 μl, 0.11 mmol) is added followed by diphenylchlorophosphate (19.9 μl, 0.10 mmol), and the resulting solution is stirred at 0°C for 55 min. Diisopropylethylamine (17.7 μl, 0.10 mmol) is added by syringe followed by p-nitrobenzyloxycarbonylaminoethanethiol (26.6 mg, 0.104 mmol), and the reaction is stirred for 2.5 hr. The reaction is then added to 20 ml ethylacetate-10 ml water. After separation of the layers, the organic layer is extracted with 8 ml 0.1M $KH_2PO_4$, 8 ml 0.1M $K_2HPO_4$, 2 × brine, dried over magnesium sulfate, filtered and concentrated *in vacuo* to 65 mg yellow foam. Chromatography on 3—1000 μ silica gel GF plates (ethylacetate) provides upon extraction (ethylacetate) of the desired UV band, concentration to an oil, addition of methylene chloride (drying over magnesium sulfate), filtration, and reconcentration *in vacuo*, 31 mg of the title compound as a pale yellow foam.

IR ($CHCl_3$) μ 5.64, 5.81 (br)

M.S. m/e 600 ($M^+$), 556, 301

NMR (300MHz, $CDCl_3$) δ 1.24 (S, $CH_3$), 1.27 (d, J=6Hz, $C\underline{H}_3CHOH$), 3.15 (d for H's with J=9Hz on top of m from 2.94—3.2 for $-SC\underline{H}_2CH_2NH-$), 3.39—3.52 (m, $-SCH_2C\underline{H}_2-NH-$), 4.15 (center of m, $CH_3C\underline{H}OH$), 4.31 (brt, J=9Hz, $H_5$), 5.20 (s, $NHCO_2C\underline{H}_2$ arom), 5.38 (center of two widely spaced d, noneq. methylene protons of ester), 7.50—8.27 (series of peaks, aromatics).

(±)-6-methylthienamycin

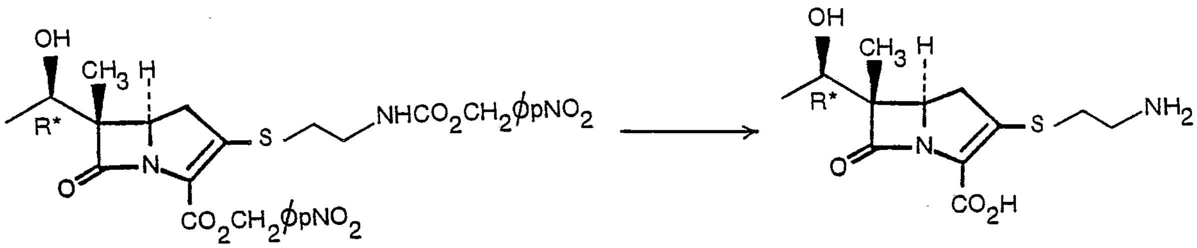

To 5.3 mg p-nitrobenzyl 6 - (1 - hydroxyethyl) - 6β - methyl - 3 - [2 - p - nitrobenzyloxycarbonyl)amino]ethylthio - 1 - azabicyclo[3.2.0]hept - 2 - en - 7 - one - 2 - carboxylate is added 500 µl distilled THF, 500 µl absolute EtOH, 330 µl DI-water, 33 µl MOPS buffer, 6.9 mg platinum oxide, and two glass beads. The reaction mixture is placed in a Parr shaker, evacuated and covered with $N_2$ alternatively (3X), evacuated and covered with 50 psi $H_2$. After cooling in an ice bath, the reaction mixture is centrifuged, and the supernatant is filtered through a small plug of cotton into a cold centrifuge tube. The residual catalyst is washed (3X) with 15 drops DI—$H_2O$ and centrifuged; the supernatants are added to the original filtrate which is finally extracted with ethylacetate (3 × 1 ml). The aqueous layer is briefly placed on an aspirator to remove residual organic solvents and then applied to a small column of XAD-2 resin (~7 ml vol) packed and eluted with DI—$H_2O$. After the first 2.5 ml, the next 30 ml eluant contains the product. The above sequence is repeated on 5.4 mg and 5.0 mg additional starting material.

The combined aqueous solutions are concentrated *in vacuo* without heat to 3 ml volume. The solution is passed portionwise through a semi-prep (9.5 ml void volume) µ-Bondapak-$C_{18}$ HPLC column (3% THF/DI—$H_2O$), flow rate-2 ml/min, 254 mµ filter), and the major peak is collected. Concentration, as above, afforded a solution of 2.1 mg of the title compound having a hydroxylamine-quenchable $UV_{max}$ at 297 mµ, essentially no electrophoretics mobility in pH7 phosphate buffer (1500V—30 min), and a clean HPLC trace (retention time ~ 6.5 min).

Example 14

Following the procedure of Example 2, the azetidinones of Table I are obtained when the basic procedure of Example 2 is modified according to the remarks entered in Table I.

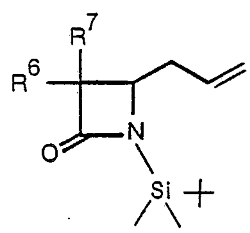

TABLE I

| Compound | $R^6$ | $R^7$ | Remarks |
|---|---|---|---|
| 1.) | $(CH_3)_2CH$ | H | As in Example 2, but substituting equivalent amount of isopropyl iodide for acetaldehyde. |
| 2.) | $CH_3$ | H | As in Example 2, but using an equivalent amount of methyl iodide for acetaldehyde. |
| 3.) | $HOCH_2$ | $CH_3$ | As in Example 2, but using compound 2., and excess formaldehyde introduced as a gas just above surface of stirred solution. |
| 4.) | 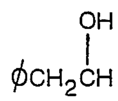 ϕ = phenyl | H | As in Example 2, but using as equivalent amount of phenyl acetaldehyde for acetaldehyde. |
| 5.) | 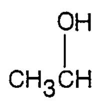 | $CH_3$ | Using the procedure of Example 2 upon compound 2 of Table I. |
| 6.) | $ϕCH_2$ | H | As in Example 2, but substituting benzylbromide for acetaldehyde. |
| 7.) | 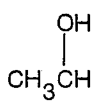 | $ϕCH_2$ | As in Example 2, but using compound 6 as substrate. |
| 8.) | 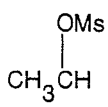 Ms = mesyl | H | Obtained from the product of Example 2 and methanesulfonyl chloride and triethylamine in methylene chloride at 0°C. |
| 9.) | 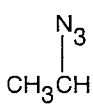 | H | Obtained from compound 8 on treatment with $LiN_3$ in DMF at 60°C. |
| 10.) | 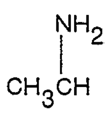 | H | Obtained from compound 9 by reduction with $H_2S$ and $Et_3N$ in $CH_2Cl_2$. |
| 10a.) | 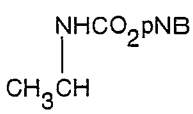 | H | Obtained from compound 9 on treatment with $ClCO_2pNB$ and DMPA in $CH_2Cl_2$ at 0°C. |
| 11.) | 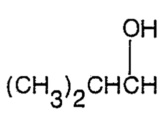 | H | As in Example 2, but substituting isobutyraldehyde for acetaldehyde. |

TABLE I (Continued)

| Compound | $R^6$ | $R^7$ | Remarks |
|---|---|---|---|
| 12.) | 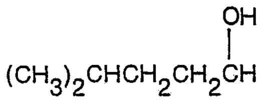 | H | As in Example 2, but substituting 5-methyl valeraldehyde for acetaldehyde. |
| 13.) | 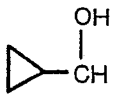 | H | As in Example 2, but substituting cyclopropane carboxaldehyde for acetaldehyde. |
| 14.) | 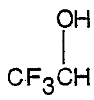 | H | As in Example 2, but substituting trifluoroacetaldehyde for acetaldehyde. |
| 15.) | 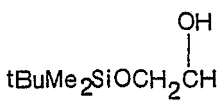 | H | As in Example 2, but substituting t-butyldimethylsilyloxyacetaldehyde for acetaldehyde. |
| 16.) | $HOCH_2CH_2$ | H | As in Example 2, but substituting oxirane for acetaldehyde. |
| 17.) | 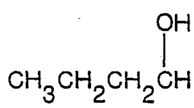 | H | As in Example 2, but substituting butyraldehyde for acetaldehyde. |
| 18.) | 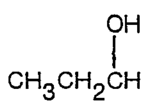 | H | As in Example 2, but substituting propionaldehyde for acetaldehyde. |
| 19.) | 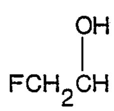 | H | As in Example 2, but substituting fluoroacetaldehyde for acetaldehyde. |
| 20.) | 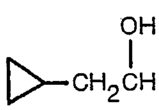 | H | As in Example 2, but substituting cyclopropylacetaldehyde for acetaldehyde. |
| 21.) | $CH_3CH_2$ | H | As in Example 2, but substituting ethyliodide for acetaldehyde. |
| 22.) | $CH_3$ | $CH_3$ | As in Example 2, but using compound 2 and substitute methyliodide for acetaldehyde. |
| 23.) | 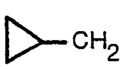 | H | As in Example 2, but substituting cyclopropylmethylbromide for acetaldehyde. |
| 24.) | $HOCH_2CH_2$ | $CH_3$ | As in Example 2, but using compound 2 and substitute oxirane for acetaldehyde. |

TABLE I (Continued)

| Compound | $R^6$ | $R^7$ | Remarks |
|---|---|---|---|
| 25.) | 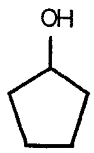 | H | As in Example 2, but using cyclopentanone instead of acetaldehyde. |
| 25a.) | 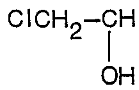 | H | As in Example 3B and 4, but using an equivalent amount of chloroacetylimidazole instead of acetylimidazole. |
| 25b.) | 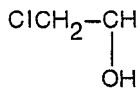 | $CH_3$ | As in Example 14, Table I, No. 25a, but starting with azetidinone of Example 14, Table I, No. 3. |
| 26.) | 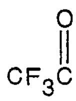 | H | As in Example 3B, but using ethyl trifluorothiolacetate instead of N-acetylimidazole. |
| 27.) | 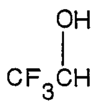 | H | As in Example 4, but substituting product No. 26, Table I, Example 14, and using sodium borohydride as reductant. |
| 28.) | 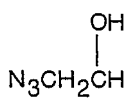 | H | As in Example 2, but using azidoacetaldehyde instead of acetaldehyde. |
| 29.) | 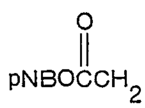 | H | As in Example 2, but substituting p-nitrobenzyl bromoacetate for acetaldehyde. |
| 30.) | 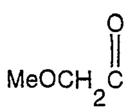 | H | As in Example 3B, but substituting N-methoxyacetyl imidazole for N-acetyl imidazole. |
| 31.) | 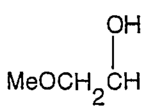 | H | Obtained by employing the procedure of Example 4 on compound No. 30, Table I, Example 14. |
| 32.) | 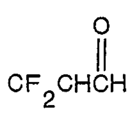 | H | As in Example 3B, but substituting ethyl difluorothiolacetate for N-acetylimidazole. |
| 33.) | 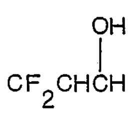 | H | Obtained from No. 32, above, using the procedure of Example 4, but substituting sodium borohydride as the reducing agent. |

TABLE I (Continued)

| Compound | $R^6$ | $R^7$ | Remarks |
|---|---|---|---|
| 34.) | pNBOCOCH$_2$<br>‖<br>O | $CH_3$ | Obtained from No. 3, Table I, Example 14, with p-nitrobenzyl chloroformate and 4-dimethylaminepyridine in methylene-chloride. |
| 35.) | O<br>‖<br>pNBOCOCH$_2$CH$_2$ | H | Obtained from No. 16, Table I, Example 14, by reaction with p-nitrobenzyl chloroformate and triethylamine in methylene chloride. |
| 36.) | O<br>‖<br>pNBOCOCH$_2$CH$_2$ | $CH_3$ | Obtained from No. 24, Table I, Example 14, as described for the preceding compound No. 35. |
| 37.) | HOCH$_2$ | H | As in Example 2, but using excess formaldehyde instead of acetaldehyde. |
| 38.) | pNBOCOCH$_2$<br>‖<br>O | H | Obtained from compound 36, above, and p-nitrobenzyl-chloroformate in methylene chloride containing 4-dimethylaminopyridine. |

Example 15

Following the foregoing Examples and text, particularly Example 9, the representative intermediates of the present invention are obtained when the indicated substitution from Example 14 is made into the scheme of Example 9.

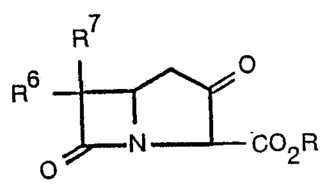

TABLE II

R = pNB (p-nitrobenzyl)

| Compound | $R^6$ | $R^7$ | Remarks |
|---|---|---|---|
| 1.) | $(CH_3)_2CH$ | H | |
| 2.) | $CH_3$ | H | |
| 3.) | 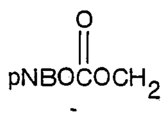 | $CH_3$ | The primary alcohol, No. 3, Table I, Example 14, is protected as shown by reacting with an equivalent amount of $ClCO_2pNB$ in the presence of DMAP (dimethylamino-propane) in methylene chloride. |
| 4.) | 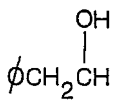 φ = phenyl | H | |
| 5.) | 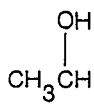 | $CH_3$ | |
| 6.) | $\phi CH_2$ | H | |
| 7.) | 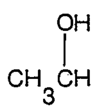 | $\phi CH_2$ | |
| 8.) | 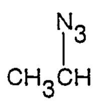 | H | |
| 9.) | 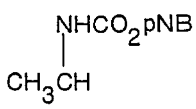 | H | |
| 10.) | 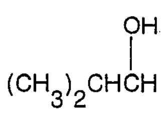 | H | |
| 11.) | 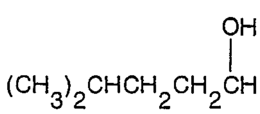 | H | |
| 12.) | 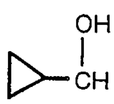 | H | |
| 13.) | 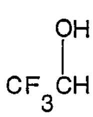 | H | |

TABLE II (Continued)

| Compound | $R^6$ | $R^7$ | Remarks |
|---|---|---|---|
| 14.) | 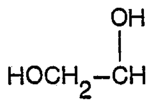 | H | |
| 15.) | 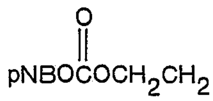 | H | Protected as described for No. 3, Table II, Example 15. |
| 16.) | 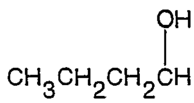 | H | |
| 17.) | 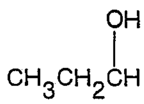 | H | |
| 18.) | 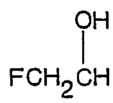 | H | |
| 19.) | 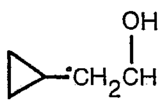 | H | |
| 20.) | $CH_3CH_2$ | H | |
| 21.) | $CH_3$ | $CH_3$ | |
| 22.) | 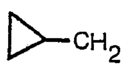 | H | |
| 23.) | 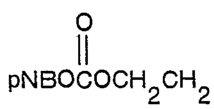 | $CH_3$ | Protected as described for No. 3, Table II, Example 15. |
| 24.) | 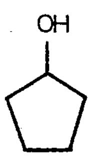 | H | |
| 25.) | 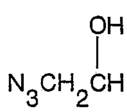 | H | |
| 25a.) | 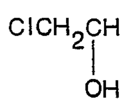 | H | |
| 25b.) | 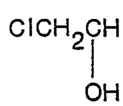 | $CH_3$ | |

TABLE II (Continued)

| Compound | $R^6$ | $R^7$ | Remarks |
|---|---|---|---|
| 26.) | 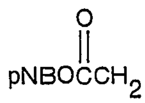 | H | |
| 27.) | 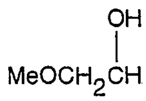 | H | |
| 28.) | 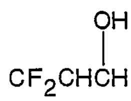 | H | |
| 29.) | 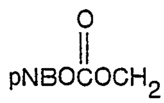 | H | |

Example 16

Following the foregoing Examples and text, the following compounds are prepared. In the following Table, the resulting compounds are taken from starting materials which are made available by the foregoing text and examples — particularly Table II of Example 15. The column labelled "Remarks and Reagents" annotates the established procedure where necessary to obtain the indicated compound. In most instances the compounds are deblocked according to the procedure described in Example 12. However, when the $SR^8$ side chain does not contain a basic function, the final product I is more conveniently isolated as the sodium salt (M=Na); which result is facilitated by conducting the deblocking in a slight excess of $NaHCO_3$. In any event, when either $R^6$ or $R^7$ bears a basic group, the final product I is most conveniently isolated as the free acid (M=H), rather than the sodium salt. It should be noted that compounds designated as "free acids" in reality are isolated as inner salts as a consequence of their zwitterionic nature.

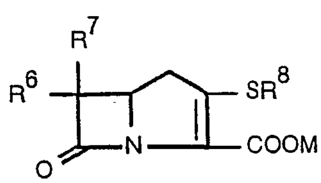

M = H, Na

TABLE III

| Compound | $R^6$ | $R^7$ | $R^8$ | Remarks, Reagents |
|---|---|---|---|---|
| 1.) | $(CH_3)_2CH$ | H | ϕ | As in Example 11, but substituting HSϕ for $HSCH_2CH_2NHCO_2$pBN. Deblock as described in Example 12 and isolate product as Na salt. M = Na. |
| 2.) | $CH_3$ | H | $CH_2$ϕ | $HSCH_2$ϕ; M = Na. |
| 3.) | $HOCH_2$ | $CH_3$ | $CH_2CH_2CH_2NH_2$ | $HSCH_2CH_2CH_2NHCO_2$pNB ; M = H. |
| 4.) | OH<br>\|<br>ϕ$CH_2CH$<br>ϕ = phenyl | H | $CH_2C(CH_3)_2NH_2$ | $HSCH_2C(CH_3)_2NHCO_2$pNB; M = H. |
| 5.) | OH<br>\|<br>$CH_3CH$ | $CH_3$ | $CH_2CH_2NH_2$ | M = H. |
| 6.) | OH<br>\|<br>$CH_3CH$ | OH<br>\|<br>$CH_3CH$ | $CH_2CH_2NH_2$ | M = H. |
| 7.) | OH<br>\|<br>$CH_3CH$ | ϕ$CH_2$ | $CH_2CH_2N(CH_3)_2$ | $HSCH_2CH_2N(CH_3)_2$; M = H. |
| 8.) | $NH_2$<br>\|<br>$CH_3CH$ | H | $CH_2CH_2NH_2$ | M = H. |

TABLE III (Continued)

| Compound | $R^6$ | $R^7$ | $R^8$ | Remarks, Reagents |
|---|---|---|---|---|
| 9.) | 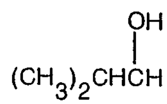 | H | 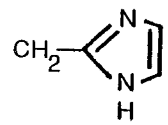 | 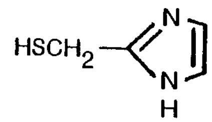 ; M = Na |
| 10.) | 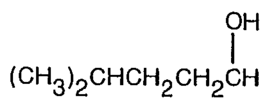 | H | 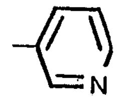 | 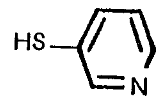 ; M = H |
| 11.) | 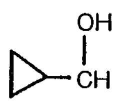 | H | 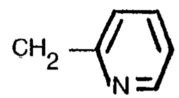 | 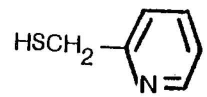 ; M = H |
| 12.) | 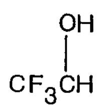 | H | $CH_2CH_2NH_2$ | M = H |
| 13.) | 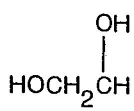 | H | $CH_2CH_2NH_2$ | M = H |

TABLE III (Continued)

| Compound | $R^6$ | $R^7$ | $R^8$ | Remarks, Reagents |
|---|---|---|---|---|
| 14.) | $HOCH_2CH_2$ | H | 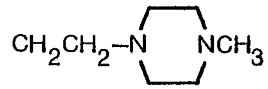 | 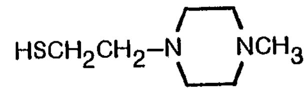 ; M= H |
| 15.) | 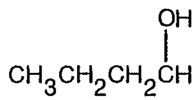 | H | 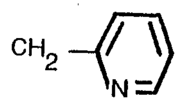 | 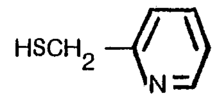 ; M = H |
| 16.) | 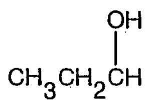 | H | 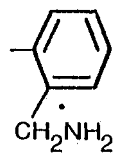 | 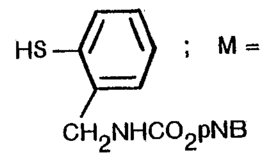 ; M = H |
| 17.) | 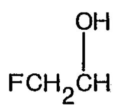 | H | $CH_2CH_2NH_2$ | |
| 18.) | 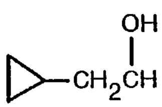 | H | $CH_2CH_2CO_2H$ | $HSCH_2CH_2CO_2pNB$ ; Product isolated as disodium salt. |
| 19.) | $CH_3CH_2$ | H | $CH_2CH_2NH_2$ | |

TABLE III (Continued)

| Compound | $R^6$ | $R^7$ | $R^8$ | Remarks, Reagents |
|---|---|---|---|---|
| 20.) | $CH_3$ | $CH_3$ | 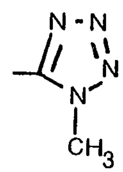 | 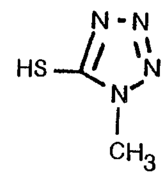 ; M = Na |
| 21.) | 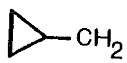 | H | $CH_2CH_2OH$ | $HSCH_2CH_2OH$ ; M = Na. |
| 22.) | $HOCH_2CH_2$ | $CH_2$ | $CH_2CH_2CH_2CH_2NH_2$ | $HSCH_2CH_2CH_2CH_2NHO_2pNB$ ; M = H. |
| 23.) | 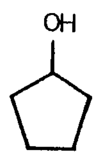 | H | $CH_2C(CH_3)_2CH_2NH_2$ | $CH_2C(CH_3)_2CH_2NHCO_2pNB$ ; M = H. |
| 24.) | 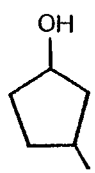 | H | $CH_2CH_2NH_2$ | M′ = Na. |
| 25.) | 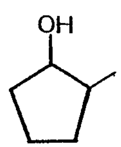 | H | $CH_2CH_2NH_2$ | M = Na. |

TABLE III (Continued)

| Compound | $R^6$ | $R^7$ | $R^8$ | Remarks, Reagents |
|---|---|---|---|---|
| 26.) | 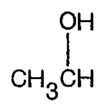 | H | 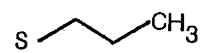 | 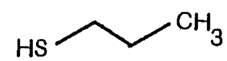 ; M = Na |
| 27.) | ,, | H | 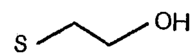 | 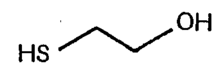 ; M = Na |
| 28.) | ,, | H | 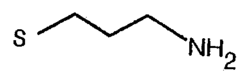 | 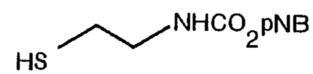 ; M = H |
| 29.) | ,, | H | 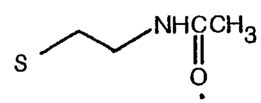 | 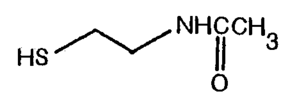 ; M = Na |
| 30.) | ,, | H | 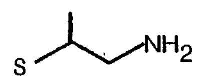 | 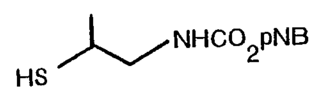 ; M = H |
| 31.) | ,, | H | 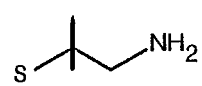 | 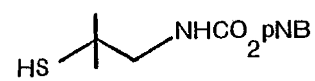 ; M = H |

TABLE III (Continued)

| Compound | $R^6$ | $R^7$ | $R^8$ | Remarks, Reagents |
|---|---|---|---|---|
| 32.) | 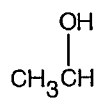 | H | 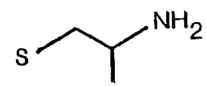 | 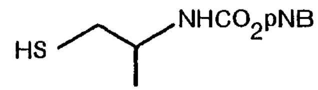 ; M = H |
| 33.) | ,, | H | 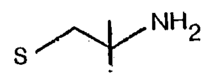 | 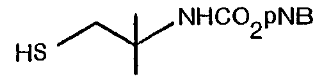 ; M = H |
| 34.) | ,, | H | 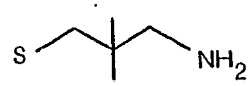 | 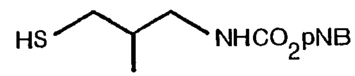 ; M = H |
| 35.) | ,, | H | 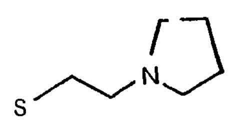 | 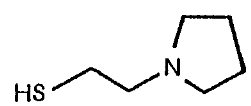 ; M = H |
| 36.) | ,, | H | 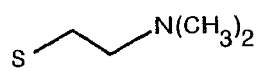 | 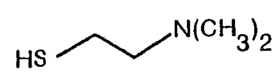 ; M = H |
| 37.) | ,, | H | 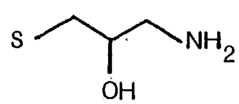 | 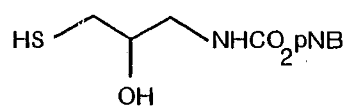 ; M = H |

TABLE III (Continued)

| Compound | $R^6$ | $R^7$ | $R^8$ | Remarks, Reagents |
|---|---|---|---|---|
| 38.) | 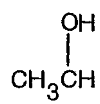 | H | 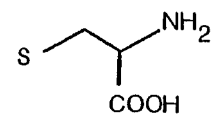 | 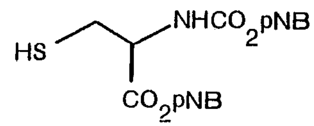 ; M = H |
| 39.) | ,, | H | 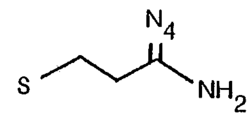 | 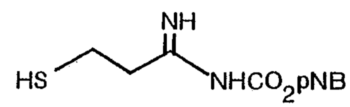 ; M = H |
| 40.) | ,, | H | 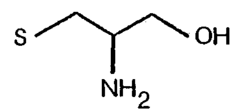 | 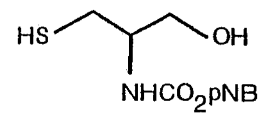 ; M = H |
| 41.) | ,, | H | 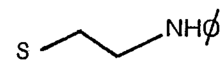 | M = H |
| 42.) | ,, | H | 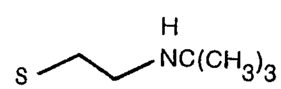 | 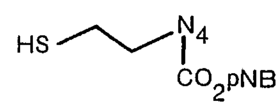 M = H |
| 43.) | ,, | H | S$\phi$ | M = Na |

TABLE III (Continued)

| Compound | $R^6$ | $R^7$ | $R^8$ | Remarks, Reagents |
|---|---|---|---|---|
| 44.) | 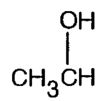 | H | 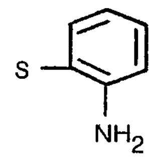 | 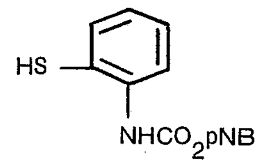 M = H |
| 45.) | ,, | H | 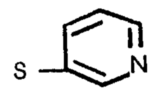 | 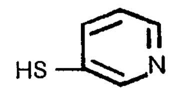 ; M = H |
| 46.) | ,, | H | 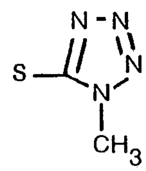 | M = Na |
| 47.) | ,, | H | 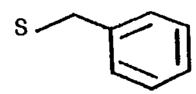 | M = Na |
| 48.) | ,, | H | 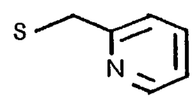 | M = H |

TABLE III (Continued)

| Compound | $R^6$ | $R^7$ | $R^8$ | Remarks, Reagents |
|---|---|---|---|---|
| 49.) | 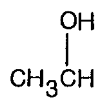 | H | 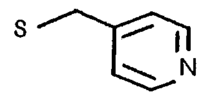 | M = H |
| 50.) | ,, | H | 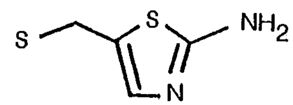 | 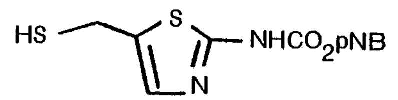 ; M = H |
| 51.) | ,, | H | 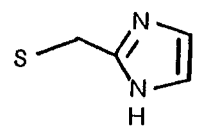 | M = Na |
| 52.) | ,, | H | 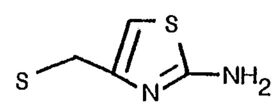 | 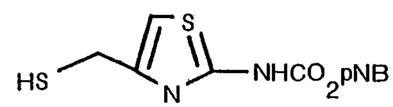 M = H |
| 53.) | ,, | H | 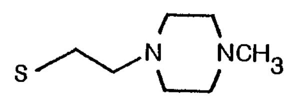 | M = H |

TABLE III (Continued)

| Compound | $R^6$ | $R^7$ | $R^8$ | Remarks, Reagents |
|---|---|---|---|---|
| 54.) | 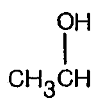 | H | 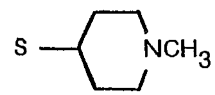 | M = H |
| 55.) | ,, | H | 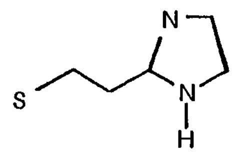 | 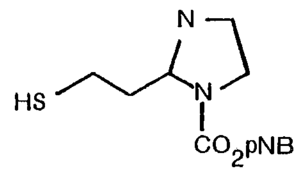 ; M = H |
| 56.) | ,, | H | 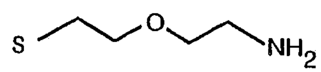 | 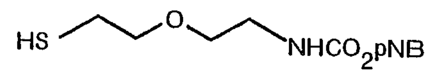 M = H |
| 57.) | ,, | H | 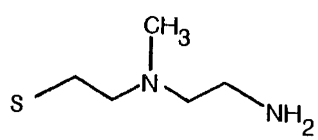 | 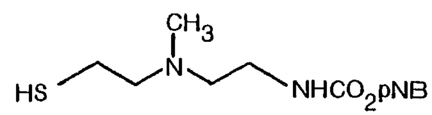 M = H |

TABLE III (Continued)

| Compounds | |
|---|---|
| 58–89 | Compounds 58–89 correspond sequentially to compounds 26–57, above, except that the value for $R^6$ is taken as $CH_3CH_2$ rather than the $CH_3C(OH)H$ of Compounds 26–57. |
| 90–121 | Compounds 90–121 correspond sequentially to compounds 26–57, above, except that the value for $R^6$ is taken as $Cl_2CH\overset{OH}{\overset{\vert}{C}}H$ rather than the $CH_3C(OH)H$ of Compounds 26–57. |
| 122–153 | Compounds 122–153 correspond sequentially to compounds 26–57, above, except that the value for $R^6$ is taken as $CF_3\overset{OH}{\overset{\vert}{C}}H$ rather than the $CH_3C(OH)H$ of Compounds 26–57. |
| 154–185 | Compounds 154–185 correspond sequentially to compounds 26–57, above, except that the value for $R^6$ is taken as $HOCH_2\overset{OH}{\overset{\vert}{C}}H$ rather than the $CH_3C(OH)H$ of Compounds 26–57. |
| 186–217 | Compounds 186–217 correspond sequentially to compounds 26–57, above, except that the value for $R^6$ is taken as $ClCH_2\overset{OH}{\overset{\vert}{C}}H$ rather than the $CH_3C(OH)H$ of Compounds 26–57. |
| 218–249 | Compounds 218–249 correspond sequentially to compounds 26–57, above, except that the value for $R^6$ is taken as $CH_3CH_2\overset{OH}{\overset{\vert}{C}}H$ rather than the $CH_3C(OH)H$ of Compounds 26–57. |
| 250–281 | Compounds 250–281 correspond sequentially to compounds 26–57, above, except that the value for $R^6$ is taken as 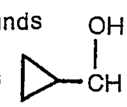 rather than the $CH_3C(OH)H$ of Compounds 26–57. |
| 282–313 | Compounds 282–313 correspond sequentially to compounds 26–57, above, except that the value for $R^6$ is taken as $H_2NCH_2\overset{OH}{\overset{\vert}{C}}H$ rather than the $CH_3C(OH)H$ of Compounds 26–57. |
| 314–345 | Compounds 314–345 correspond sequentially to compounds 26–57, above, except that the value for $R^6$ is taken as $CF_2\overset{OH}{\overset{\vert}{H}}C-$ rather than the $CH_3C(OH)H$ of Compounds 26–57. |

TABLE III (Continued)

| Compounds | |
|---|---|
| 346–377 | Compounds 346–377 correspond sequentially to compounds 26–57, above, except that the value for $R^6$ is taken as $HOCH_2$ rather than the $CH_3C(OH)H$ of Compounds 26–57. |
| 378–409 | Compounds 378–409 correspond sequentially to compounds 26–57. above, except that the value for $R^6$ is taken as $HO_2CCH_2$ rather than the $CH_3C(OH)H$ of Compounds 26–57. |
| 410–441 | Compounds 410–441 correspond sequentially to compounds 26–57, above, except that the value for $R^6$ is taken as $CH_3OCH_2\overset{\overset{OH}{\vert}}{C}H$ rather than the $CH_3C(OH)H$ of Compounds 26–57. |
| 442–473 | Compounds 442–473 correspond sequentially to compounds 26–57, above, except that the value for $R^6$ is taken as $(CH_3)_3CCH_2\overset{\overset{OH}{\vert}}{C}H$ rather than the $CH_3C(OH)H$ of Compounds 26–57. |

The compounds of Examples 1 to 12 and those of 26 to 57 are not subject of the claimed matter.

**Claims**

1. 4-(3-Carboxy-2-oxopropyl)-azetidino-2-ones selected from

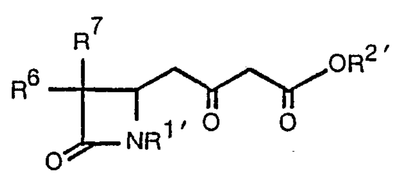

and

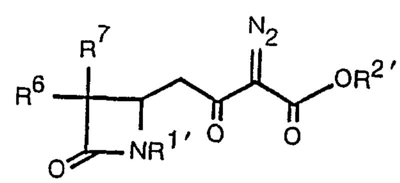

wherein $R^{1'}$ and $R^{2'}$ are hydrogen or a readily removable protecting group; and wherein $R^6$ and $R^7$ are independently selected from hydrogen; substituted and unsubstituted: alkyl, alkenyl, and alkynyl, having from 1—10 carbon atoms; cycloalkyl, cycloalkylalkyl, and alkylcycloalkyl, having 3—6 carbon atoms in the cycloalkyl ring and 1—6 carbon atoms in the alkyl moieties; phenyl; aralkyl, aralkenyl, and aralkynyl wherein the aryl moiety is phenyl and the aliphatic portion has 1—6 carbon atoms; heteroaryl, heteroaralkyl, heterocyclyl and heterocyclylalkyl; wherein the substituent or substituents relative to the above-named radicals are selected from

chloro, bromo, fluoro,

$-OH$, $-NR^1R^2$,

$-OR^1$, $-NH_2$,

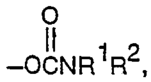

$-NHR^1$

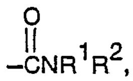

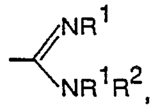

$-SO_2NR^1R^2$,

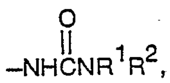

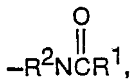

$-CO_2H$ ,

$-CO_2R^1$,

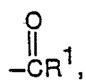

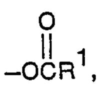

$-SH$ ,

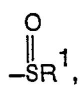

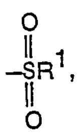

$-CN$ , and

$-N_3$

wherein, relative to the above listed substituents on $R^6$, and $R^7$, the groups $R^1$ and $R^2$ are independently selected from: hydrogen, alkyl, alkenyl, and alkynyl, having from 1—10 carbon atoms; cycloalkyl, cycloalkylalkyl, and alkylcycloalkyl, having 3—6 carbon atoms in the cycloalkyl ring and 1—6 carbon atoms in the alkyl moieties; phenyl; aralkyl, aralkenyl, and aralkynyl wherein the aryl moiety is phenyl and the aliphatic portion has 1—6 carbon atoms; heteroaryl, heteroaralkyl, heterocyclyl and heterocyclylalkyl; and wherein

the above-named heterocyclic moieties defined for $R^6$, $R^7$, $R^1$ and $R^2$ are derived from the following alkylating or acylating agents

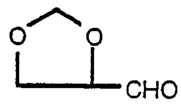

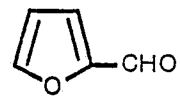

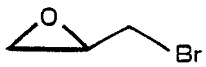

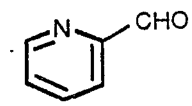

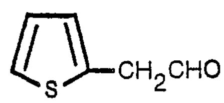,

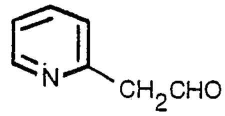

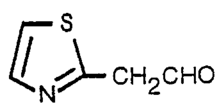,

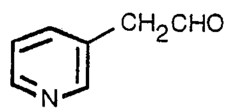

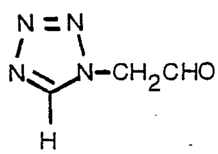,

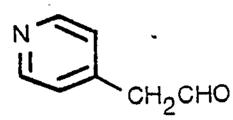

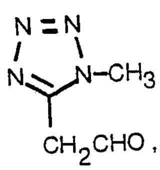,

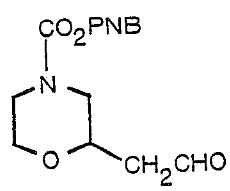

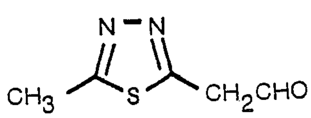,

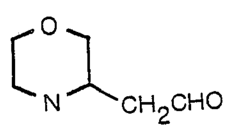,

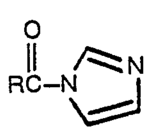 ; R = $CH_3$, $ClCH_2$, $CH_3CH_2$, $N_3CH_2$, $CH_3OCH_2$,

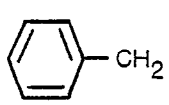, 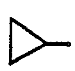, 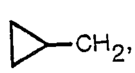, 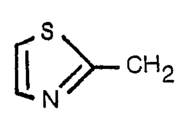,

and wherein the alkyl moieties associated with said heterocyclic moieties have 1—6 carbon atoms; $R^6$ and $R^7$ are not both hydrogen at the same time; when $R^6/R^7$ is hydrogen, then $R^7/R^6$ is not

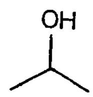

or

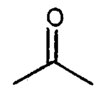

.

2. A compound according to Claim 1 wherein $R^{1'}$ is hydrogen or a triorganosilyl group.

3. A compound according to Claim 2 wherein $R^{1'}$ is hydrogen or a trialkylsilyl group wherein each alkyl moiety has from 1—6 carbon atoms.

4. A compound according to Claims 1 wherein $R^6$ and $R^7$ are independently selected from: hydrogen; substituted and unsubstituted: alkyl, alkenyl and alkynyl having from 1—10 carbon atoms, cycloalkyl, cycloalkylalkyl and alkylcycloalkyl, having 3—6 carbon atoms in the cycloalkyl ring and 1—6 carbon atoms in the alkyl moieties; phenyl; aralkyl, aralkenyl, and aralkynyl wherein the aryl moiety is phenyl and the aliphatic portion has 1—6 carbon atoms; wherein the substituent or substituents relative to the above-named radicals are selected from:

chloro, bromo, fluoro,

$-OH$,

$-OR^1$,

$-O\overset{O}{\overset{\|}{C}}NR^1R^2$,

$-\overset{O}{\overset{\|}{C}}NR^1R^2$,

$-NR^1R^2$,

$-NH_2$,

$-NHR^1$,

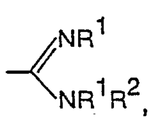

$-SO_2NR^1R^2$,

$-NH\overset{O}{\overset{\|}{C}}NR^1R^2$,

$-R^2N\overset{O}{\overset{\|}{C}}R^1$,

$-CO_2H$ ,

$-CO_2R^1$,

$-\overset{O}{\overset{\|}{C}}R^1$,

$-O\overset{O}{\overset{\|}{C}}R^1$,

$-SH$ ,

$-\overset{O}{\overset{\|}{S}}R^1$,

$-\underset{\underset{O}{\|}}{\overset{\overset{O}{\|}}{S}}R^1$,

$-CN$ , and

$-N_3$

wherein, relative to the above listed substituents on $R^6$, $R^7$, the groups $R^1$ and $R^2$ are independently selected from: hydrogen, alkyl, alkenyl and alkynyl, having from 1—10 carbon atoms; cycloalkyl, cycloalkylalkyl and alkylcycloalkyl, having 3—6 carbon atoms in the cycloalkyl ring and 1—6 carbon atoms in the alkyl moieties, phenyl; aralkyl, aralkenyl, and aralkynyl wherein the aryl moiety is phenyl and the aliphatic portion has 1—6 carbon atoms.

5. A compound according to Claims 1, 2, 3 or 4 wherein $R^7$ is hydrogen or methyl.

6. A compound according to Claim 3 wherein $R^6$ is selected from: substituted and unsubstituted: alkyl, alkenyl and cycloalkylalkyl wherein the substituent or substituents are selected from hydroxyl, alkoxyl having from 1—6 carbon atoms, phenoxy, amino, and carboxy.

7. A compound according to Claims 5 or 6 wherein $R^6$ is selected from alkyl, cycloalkylalkyl, alkyl substituted by one or more hydroxyl groups, or cycloalkylalkyl substituted by one or more hydroxyl groups.

8. A compound according to Claims 5, 6 or 7 wherein $R^7$ is hydrogen.

9. A process for preparing a compound according to Claims 1, 2, 3, 4, 5, 6, 7 or 8 comprising treating:

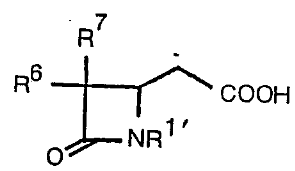

with 1,1'-carbonylimidazole followed by reacting with $(R^{2'}O_2CCH_2CO_2)_2Mg$ to yield:

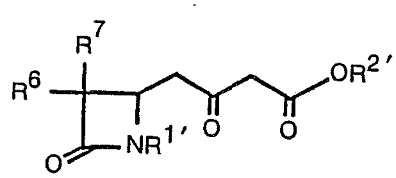

10. A process for preparing a compound according to Claims 1, 2, 3, 4, 5, 6, 7 or 8 comprising diazotizing:

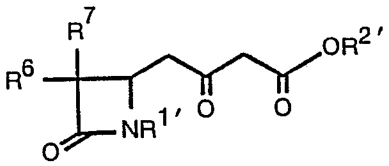

to form:

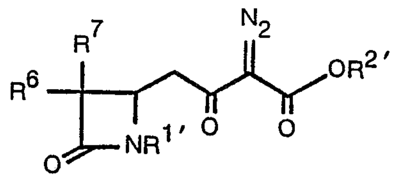

11. A compound selected from

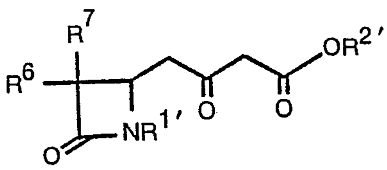

,

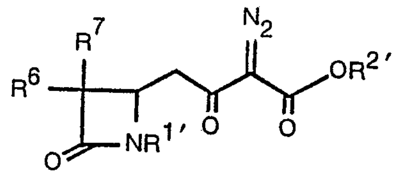

wherein $R^{1'}$ and $R^{2'}$ are independently selected from hydrogen and a readily removable protecting group; $R^7$ is hydrogen and $R^6$ is selected from $CH_3CH_2$

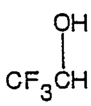

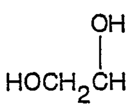

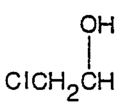

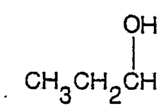

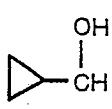

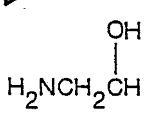

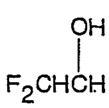

$HOCH_2$

$HO_2CCH_2$

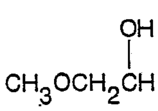

**Patentansprüche**

1. 4-(3-Carboxy-2-oxopropyl)-azetidino-2-one, ausgewählt aus

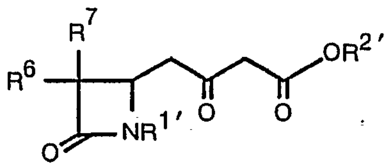

und

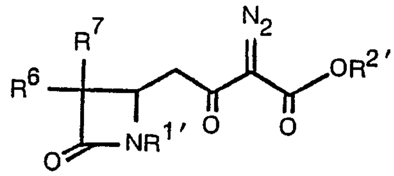

in der $R^{1'}$ und $R^{2'}$ Wasserstoff oder eine leicht entfernbare Schutzgruppe bedeuten; und in der $R^6$ und $R^7$ unabhängig voneinander ausgewählt sind aus: Wasserstoff; folgende substituierte und unsubstituierte Gruppen: Alkyl, Alkenyl und Alkinyl, mit 1 bis 10 Kohlenstoffatomen; Cycloalkyl, Cycloalkylalkyl und Alkylcycloalkyl, mit 3 bis 6 Kohlenstoffatomen im Cycloalkylring und 1 bis 6 Kohlenstoffatomen in den Alkylresten; Phenyl; Aralkyl, Aralkenyl und Aralkinyl, wobei es sich beim Arylrest um Phenyl handelt und der aliphatische Teil 1 bis 6 Kohlenstoffatome aufweist; Heteroaryl, Heteroaralkyl, Heterocyclyl und Heterocyclylalkyl; wobei der oder die Substituenten der vorstehend erwähnten Reste ausgewählt sind aus:

Chlor, Brom, Fluor

$-OH$, $-NR^1R^2$,

$-OR^1$, $-NH_2$,

$-O\overset{O}{\overset{\|}{C}}NR^1R^2$, $-NHR^1$

$-\overset{O}{\overset{\|}{C}}NR^1R^2$,

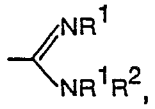

$-SO_2NR^1R^2$,

$-NH\overset{O}{\overset{\|}{C}}NR^1R^2$,

$-R^2N\overset{O}{\overset{\|}{C}}R^1$,

$-CO_2H$,

$-CO_2R^1$,

$-\overset{O}{\overset{\|}{C}}R^1$,

$-O\overset{O}{\overset{\|}{C}}R^1$,

$-SH$,

$-\overset{O}{\overset{\|}{S}}R^1$,

$-\underset{\underset{O}{\|}}{\overset{O}{\overset{\|}{S}}}R^1$,

$-CN$, und $-N_3$

wobei bezüglich der vorerwähnten Substituenten an $R^6$ und $R^7$ die Reste $R^1$ und $R^2$ unabhängig voneinander ausgewählt sind aus: Wasserstoff, Alkyl, Alkenyl und Alkinyl, mit 1 bis 10 Kohlenstoffatomen; Cycloalkyl, Cycloalkylalkyl und Alkylcycloalkyl, mit 3 bis 6 Kohlenstoffatomen im Cycloalkylring und 1 bis 6 Kohlenstoffatomen in den Alkylresten; Phenyl; Aralkyl, Aralkenyl und Aralkinyl, wobei es sich beim Arylrest um Phenyl handelt und der aliphatische Teil 1 bis 6 Kohlenstoffatome aufweist; Heteroaryl, Heteroaralkyl, Heterocyclyl und Heterocyclylalkyl; und wobei die vorerwähnten, für $R^6$, $R^7$, $R^1$ und $R^2$ definierten heterocyclischen Reste sich von den folgenden Alkylierungs- oder Acylierungsmitteln ableiten

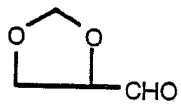
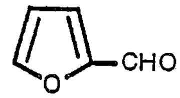
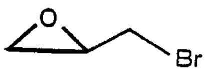
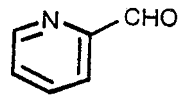
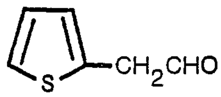 ,
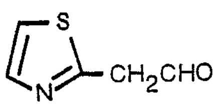 ,
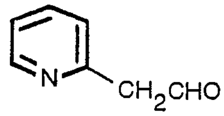
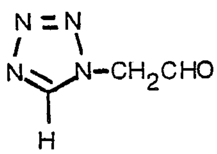 ,
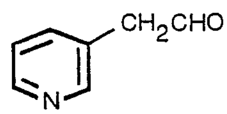
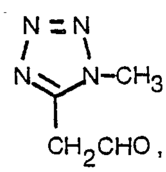 ,
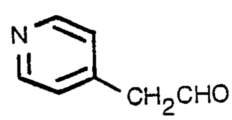
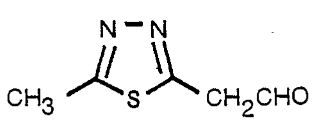 ,
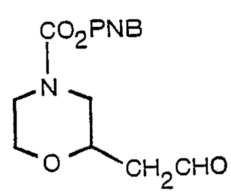
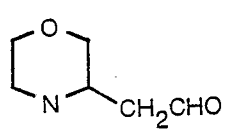 ,

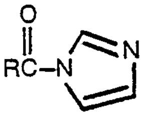 ; $R = CH_3$, $ClCH_2$, $CH_3CH_2$, $N_3CH_2$, $CH_3OCH_2$,

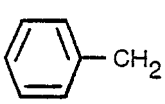 , 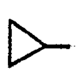 , 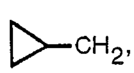 , 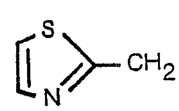 ,

und wobei die mit diesen heterocyclischen Resten verbundenen Alkylreste 1 bis 6 Kohlenstoffatome aufweisen; wobei $R^6$ und $R^7$ nicht gleichzeitig Wasserstoff bedeuten; und wobei, wenn $R^6/R^7$ Wasserstoff bedeutet, $R^7/R^6$ nicht

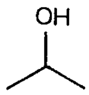 oder 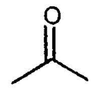 ist.

2. Verbindung nach Anspruch 1, wobei $R^{1'}$ Wasserstoff oder eine Triorganosilylgruppe bedeutet.

3. Verbindung nach Anspruch 2, wobei $R^{1'}$ Wasserstoff oder eine Trialkylsilylgruppe, in der die Alkylreste jeweils 1 bis 6 Kohlenstoffatome aufweisen, bedeutet.

4. Verbindung nach Anspruch 1, wobei $R^6$ und $R^7$ unabhängig voneinander ausgewählt sind aus: Wasserstoff; folgende substituierte und unsubstituierte Reste: Alkyl, Alkenyl und Alkinyl, mit 1 bis 10 Kohlenstoffatomen; Cycloalkyl, Cycloalkylalkyl und Alkylcycloalkyl mit 3 bis 6 Kohlenstoffatomen im Cycloalkylring und 1 bis 6 Kohlenstoffatomen in den Alkylresten; Phenyl; Aralkyl, Aralkenyl und Aralkinyl, wobei es sich beim Arylrest um Phenyl handelt und der aliphatische Teil 1 bis 6 Kohlenstoffatome aufweist; wobei der oder die Substituenten an den vorerwähnten Resten ausgewählt sind aus:

Chlor, Brom, Fluor

$-OH$,

$-OR^1$,

$-O\overset{O}{\overset{\|}{C}}NR^1R^2$,

$-\overset{O}{\overset{\|}{C}}NR^1R^2$,

$-NR^1R^2$,

$-NH_2$,

$-NHR^1$,

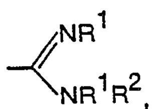,

$-SO_2NR^1R^2$,

$-NH\overset{O}{\overset{\|}{C}}NR^1R^2$,

$-R^2N\overset{O}{\overset{\|}{C}}R^1$,

$-CO_2H$ ,

$-CO_2R^1$,

$-\overset{O}{\overset{\|}{C}}R^1$,

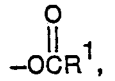

−SH ,

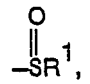

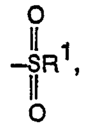

−CN , und

$-N_3$

wobei bezüglich der vorerwähnten Substituenten an $R^6$ und $R^7$ die Reste $R^1$ und $R^2$ unabhängig voneinander ausgewählt sind aus: Wasserstoff, Alkyl, Alkenyl und Alkinyl, mit 1 bis 10 Kohlenstoffatomen; Cycloalkyl, Cycloalkylalkyl und Alkylcycloalkyl, mit 3 bis 6 Kohlenstoffatomen im Cycloalkylring und 1 bis 6 Kohlenstoffatomen in den Alkylresten; Phenyl; Aralkyl, Aralkenyl und Aralkinyl, wobei es sich beim Arylrest um Phenyl handelt und der aliphatische Teil 1 bis 6 Kohlenstoffatome aufweist.

5. Verbindung nach den Ansprüchen 1, 2, 3 oder 4, wobei $R^7$ Wasserstoff oder methyl bedeutet.

6. Verbindung nach Anspruch 3, wobei $R^6$ ausgewählt ist aus: folgende substituierte und unsubstituierte Reste: Alkyl, Alkenyl und Cycloalkylalkyl, wobei der oder die Substituenten ausgewählt sind aus Hydroxyl, Alkoxy mit 1 bis 6 Kohlenstoffatomen, Phenoxy, Amino und Carboxy.

7. Verbindungen nach Anspruch 5 oder 6, wobei $R^6$ ausgewählt ist aus Alkyl, Cycloalkylalkyl, durch einen oder mehrere Hydroxylgruppen substituiertes Alkyl oder durch einen oder mehrere Hydroxylgruppen substituiertes Cycloalkylalkyl.

8. Verbindung nach Anspruch 5, 6 oder 7, wobei $R^7$ Wasserstoff bedeutet.

9. Verfahren zur Herstellung einer Verbindung nach den Ansprüchen 1, 2, 3, 4, 5, 6, 7 oder 8, umfassend die Behandlung von:

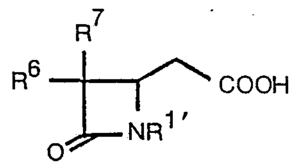

mit 1,1'-Carbonylimidazol und anschliessende Umsetzung mit $(R^2O_2CCH_2CO_2)_2Mg$ unter Bildung von:

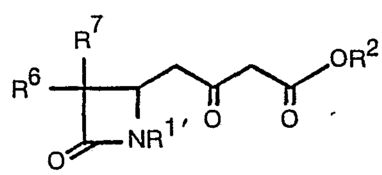

10. Verfahren zur Herstellung einer Verbindung nach den Ansprüchen 1, 2, 3, 4, 5, 6, 7 oder 8, umfassend die Diazotierung von:

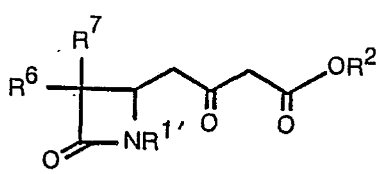

unter Bildung von:

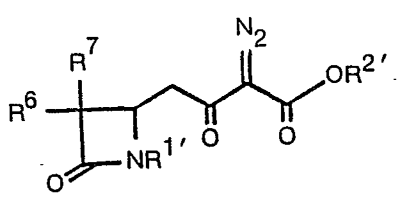

11. Verbindung ausgewählt aus:

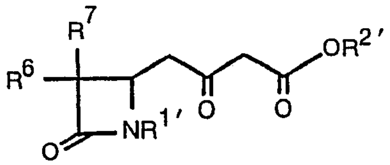

,

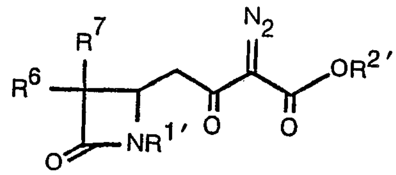

wobei $R^{1'}$ und $R^{2'}$ unabhängig voneinander ausgewählt sind aus Wasserstoff und einer leicht entfernbaren Schutzgruppe; $R^7$ Wasserstoff bedeutet und $R^6$ ausgewählt ist aus

$CH_3CH_2$

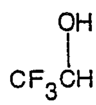

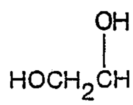

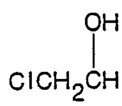

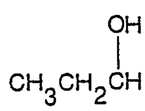

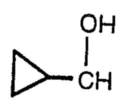

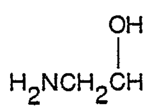

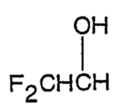

$HOCH_2$

$HO_2CCH_2$

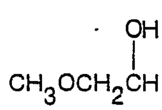

## Revendications

1. 4-(3-carboxy-2-oxopropyl)-azétidino-2-ones choisies parmi

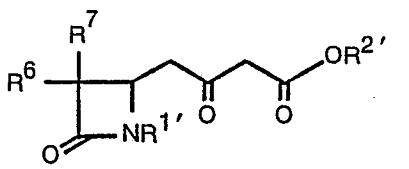

et

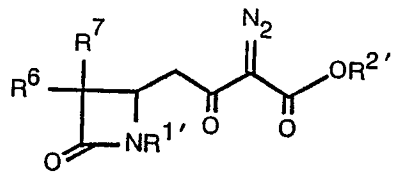

dans lesquelles $R^{1'}$ et $R^{2'}$ représentent l'hydrogène ou un groupe protecteur facile à éliminer; et dans lesquelles $R^6$ et $R^7$ sont choisis indépendamment parmi l'hydrogène; les groupes substitués et non substitués alkyle, alcényle et alcynyle contenant 1 à 10 atomes de carbone; cycloalkyle, cycloalkylalkyle et alkylcycloalkyle contenant 3 à 6 atomes de carbone dans le noyau cycloalkyle et 1 à 6 atomes de carbone dans les radicaux alkyle; phényle, aralkyle, aralcényle et aralcynyle dans lesquels le noyau aryle est un noyau phényle et la partie aliphatique contient 1 à 6 atomes de carbone; hétéroaryle, hétéroaralkyle, hétérocyclyle, hétérocyclyalkyle; dans lesquelles le ou les substituants des radicaux mentionnés ci-dessus sont choisis parmi les suivants:

chloro, bromo, fluoro,

$-OH$, $-NR^1R^2$,

$-OR^1$, $-NH_2$,

$-O\overset{O}{\overset{\|}{C}}NR^1R^2$, $-NHR^1$

$-\overset{O}{\overset{\|}{C}}NR^1R^2$,

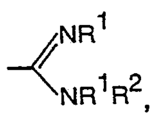

$-SO_2NR^1R^2$,

$-NH\overset{O}{\overset{\|}{C}}NR^1R^2$,

$-R^2N\overset{O}{\overset{\|}{C}}R^1$,

$-CO_2H$,

$-CO_2R^1$,

$-\overset{O}{\overset{\|}{C}}R^1$,

$-O\overset{O}{\overset{\|}{C}}R^1$,

$-SH$,

$-\overset{O}{\overset{\|}{S}}R^1$,

$-\overset{O}{\overset{\|}{\underset{\underset{O}{\|}}{S}}}R^1$,

$-CN$, et $-N_3$

dans lesquelles, pour ce qui concerne les substituants de $R^6$ et $R^7$ mentionnés ci-dessus, $R^1$ et $R^2$ sont choisis indépendamment parmi: l'hydrogène, les groupes alkyle, alcényle et alcynyle contenant de 1 à 10 atomes de carbone; cycloalkyle, cycloalkylalkyle et alkylcycloalkyle contenant 3 à 6 atomes de carbone dans le noyau cycloalkyle et 1 à 6 atomes de carbone dans les radicaux alkyle; phényle; aralkyle, aralcényle et aralcynyle dans lesquels le noyau aryle est un noyau phényle et la partie aliphatique contient 1 à 6 atomes de carbone; hétéroaryle, hétéroaralkyle, hétérocyclyle et hétérocyclylalkyle; et dans lesquelles les motifs hétérocycliques mentionnés ci-dessus en référence à $R^6$, $R^7$, $R^1$ et $R^2$ dérivent des agents alkylants ou acylants suivants:

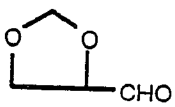

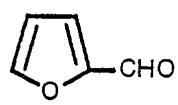

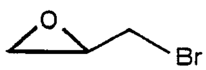

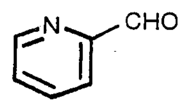

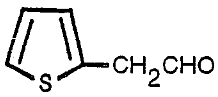,

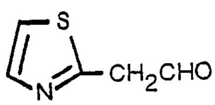,

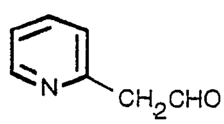

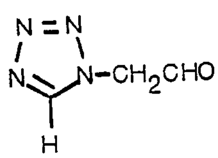,

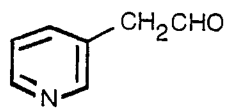

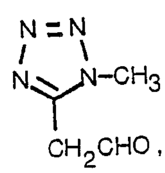,

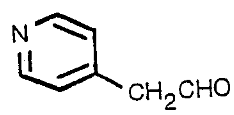

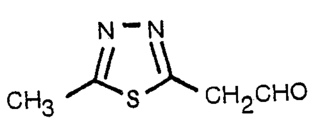,

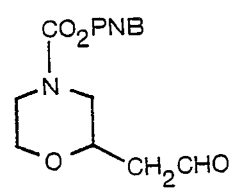

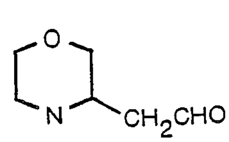,

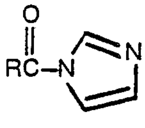 ; $R = CH_3$, $ClCH_2$, $CH_3CH_2$, $N_3CH_2$, $CH_3OCH_2$,

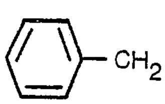, 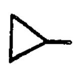, 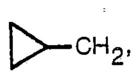, 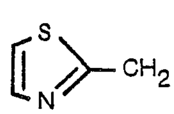,

et dans lesquelles les radicaux alkyle associés à ces motifs hétérocycliques contiennent de 1 à 6 atomes de carbone; $R^6$ et $R^7$ ne peuvent représenter tous deux l'hydrogène; lorsque $R^6/R^7$ représente l'hydrogène, $R^7/R^6$ ne peut représenter

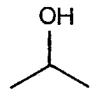

ou

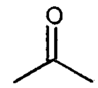

2. Un composé selon la revendication 1, dans lequel $R^{1'}$ représente l'hydrogène ou un groupe triorganosilyle.

3. Un composé selon la revendication 2, dans lequel $R^{1'}$ représente l'hydrogène ou un groupe trialkylsilyle contenant 1 à 6 atomes de carbone dans chaque radical alkyle.

4. Un composé selon la revendication 1, dans lequel $R^6$ et $R^7$ sont choisis indépendamment parmi l'hydrogène, les groupes substitués et non substitués alkyle, alcényle et alcynyle contenant 1 à 10 atomes de carbone, cycloalkyle, cycloalkylalkyle et alkylcycloalkyle contenant 3 à 6 atomes de carbone dans le noyau cycloalkyle et 1 à 6 atomes de carbone dans les radicaux alkyle; phényle; aralkyle, aralcényle et aralcynyle dans lesquels le radical aryle est un radical phényle et la partie aliphatique contient 1 à 6 atomes de carbone; dans lequel le ou les substituants des radicaux mentionnés ci-dessus sont choisis parmi les suivants:

chloro, bromo, fluoro,

$-OH$,

$-OR^1$,

$-O\overset{O}{\overset{\|}{C}}NR^1R^2$,

$-\overset{O}{\overset{\|}{C}}NR^1R^2$,

$-NR^1R^2$,

$-NH_2$,

$-NHR^1$,

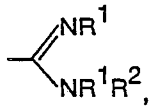,

$-SO_2NR^1R^2$,

$-NH\overset{O}{\overset{\|}{C}}NR^1R^2$,

$-R^2N\overset{O}{\overset{\|}{C}}R^1$,

$-CO_2H$,

$-CO_2R^1$,

$-\overset{O}{\overset{\|}{C}}R^1$,

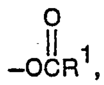

$-SH$ ,

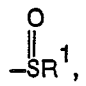

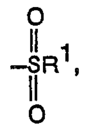

$-CN$ , et

$-N_3$

dans lequel, pour ce qui concerne les substituants énumérés ci-dessus de $R^6$, $R^7$, les substituants $R^1$ et $R^2$ sont choisis indépendamment parmi: l'hydrogène, les groupes alkyle, alcényle et alcynyle contenant 1 à 10 atomes de carbone; cycloalkyle, cycloalkylalkyle et alkylcycloalkyle contenant 3 à 6 atomes de carbone dans le noyau cycloalkyle et 1 à 6 atomes de carbone dans les radicaux alkyle, phényle; aralkyle, aralcényle et aralcynyle dans lesquels le radical aryle est un radical phényle et la partie aliphatique contient 1 à 6 atomes de carbone.

5. Un composé selon les revendication 1, 2, 3 ou 4, dans lequel $R^7$ représente l'hydrogène ou le groupe méthyle.

6. Un composé selon la revendication 3, dans lequel $R^6$ est choisi parmi les groupes substitués et non substitués alkyle, alcényle et cycloalkylalkyle, le ou les substituants étant choisis parmi les groupes hydroxy, alcoxy en $C_1$—$C_6$, phénoxy, amino et carboxy.

7. Un composé selon la revendication 5 ou 6 dans lequel $R^6$ est choisi parmi les groupes alkyle, cycloalkylalkyle, alkyle substitué par un ou plusieurs groupes hydroxy ou cycloalkylalkyle substitué par un ou plusieurs groupes hydroxy.

8. Un composé selon les revendications 5, 6 ou 7 dans lequel $R^7$ représente l'hydrogène.

9. Un procédé de préparation d'un composé selon les revendications 1, 2, 3, 4, 5, 6, 7 ou 8, qui consiste à traiter:

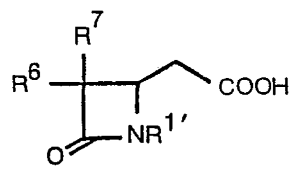

par le 1,1'-carbonylimidazole, puis à faire réagir avec $(R^{2'}O_2CCH_2CO_2)_2Mg$, ce qui donne:

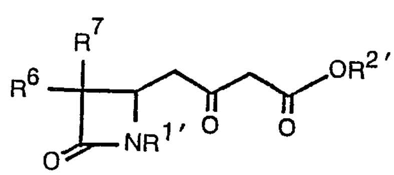

10. Un procédé de préparation d'un composé selon les revendications 1, 2, 3, 4, 5, 6, 7 ou 8 qui consiste à diazoter

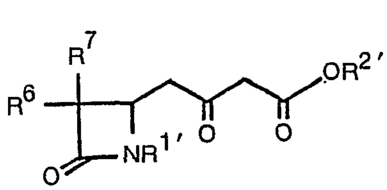

ce qui donne:

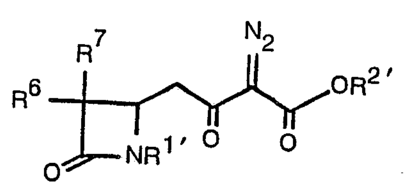

11. Un composé choisi parmi:

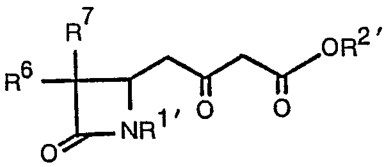 , 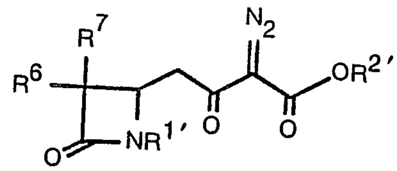

dans lequel $R^{1'}$ et $R^{2'}$ sont choisis indépendamment parmi l'hydrogène et les groupes protecteurs faciles à éliminer; $R^7$ représente l'hydrogène et $R^6$ est choisi parmi:

$CH_3CH_2$

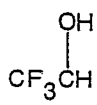

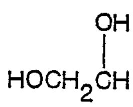

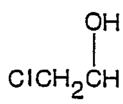

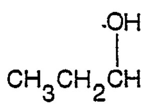

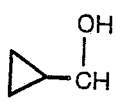

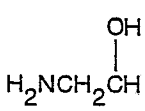

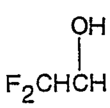

$HOCH_2$

$HO_2CCH_2$